# FASCICULE DE BREVET EUROPEEN

(11) **EP 3 528 815 B1**
(45) Date de publication et mention de la délivrance du brevet: **14.06.2023**
(21) Numéro de dépôt: 17794262.0
(22) Date de dépôt: 20.10.2017
(51) Int. Cl.: A61K 31/52, A61P 11/00, A61K 31/7064, A61P 35/00

(54) **2,6-DIAMINOPURINE POUR SON UTILISATION DANS LE TRAITEMENT OU LA PREVENTION DES MALADIES DUES A UNE MUTATION NON-SENS**
2,6-DIAMINOPURIN ZUR VERWENDUNG BEI DER BEHANDLUNG ODER VORBEUGUNG VON DURCH NONSENSE-MUTATION VERURSACHTEN KRANKHEITEN
2,6-DIAMINOPURINE FOR USE IN THE TREATMENT OR PREVENTION OF DISEASES CAUSED BY A NONSENSE MUTATION

(30) Priorité: 21.10.2016 FR 1660229
(43) Date de publication de la demande: 28.08.2019
(73) Titulaire: Muséum National D'histoire Naturelle, 75005 Paris (FR); Centre National de la Recherche Scientifique, 75016 Paris (FR); Université Lille 1 - Sciences Et Technologies, 59655 Villeneuve D'ascq Cedex (FR)
(72) Inventeur: REBUFFAT, Sylvie, 75013 Paris (FR); MAULAY-BAILLY, Christine, 75010 Paris (FR); AMAND, Séverine, 94360 Bry-Sur-Marne (FR); LEJEUNE, Fabrice, 59184 Sainghin En Weppes (FR)
(74) Mandataire: Gevers & Orès
(86) Numéro de dépôt international: PCT/EP2017/076846
(87) Numéro de publication internationale: WO 2018/073413

(56) Documents cités:
- WO-A1-2014/083327
- WO-A1-2018/073414
- WO-A2-03/004511
- WO-A2-2004/009610
- WO-A2-2016/087665
- KERSTIN NAGEL-WOLFRUM ET AL: "Targeting Nonsense Mutations in Diseases with Translational Read-Through-Inducing Drugs (TRIDs)", BIODRUGS, ADIS INTERNATIONAL LTD, NZ, vol. 30, no. 2, 1 avril 2016 (2016-04-01), pages 49-74, XP009194809, ISSN: 1173-8804, DOI: 10.1007/S40259-016-0157-6 [extrait le 2016-02-17]
- TRZASKA CAROLE ET AL: "2,6-Diaminopurine as a highly potent corrector of UGA nonsense mutations", NATURE COMMUNICATIONS, vol. 11, no. 1, 20 March 2020 (2020-03-20) , pages 1-12, XP055790585, DOI: 10.1038/s41467-020-15140-z Retrieved from the Internet: URL:http://www.nature.com/articles/s41467- 020-15140-z>

## Description

La présente invention concerne le domaine des médicaments destinés au traitement de maladies dues à une mutation non-sens. L'invention concerne en particulier une nouvelle application thérapeutique de la 2,6-diaminopurine ou d'un de ses dérivés pour le traitement des maladies dues à une mutation non-sens UGA.

Les mutations non-sens correspondent à des mutations ponctuelles qui conduisent à transformer un codon permettant l'incorporation d'un acide aminé lors de la traduction, en un codon stop (UGA, UAG ou UAA) qui provoque l'arrêt de la traduction.

Ces mutations sont à l'origine d'environ dix pourcent des cas de maladies génétiques rares, telles que la mucoviscidose, la myopathie de Duchenne, l'hémophilie, le nanisme, etc... ou fréquentes telles que certaines maladies métaboliques, neurologiques ou cancers (Mort *et al.,* 2008).

La conséquence d'une mutation non-sens est l'activation d'un mécanisme de surveillance des ARNm qui va dégrader l'ARNm porteur de la mutation non-sens. Ce mécanisme est appelé NMD (pour « nonsense-mediated mRNA decay ») et prévient la synthèse d'une protéine tronquée (Hug *et al.,* 2016 ; Kervestin and Jacobson, 2012; Popp and Maquat, 2014; Schweingruber *et al.,* 2013). Une mutation non-sens conduit par conséquent à l'absence d'expression du gène porteur de cette mutation.

Plusieurs stratégies ont été développées pour corriger les conséquences d'une mutation non-sens (Benhabiles *et al.,* 2016), notamment l'inhibition du NMD et/ou l'activation de la translecture. La translecture est un mécanisme conduisant à l'incorporation, au cours de la traduction, d'un acide aminé lorsque le ribosome atteint un codon stop prématuré. La translecture n'est pas observée sur le codon stop physiologique (*i.e.,* le codon stop terminant une phase ouverte de lecture sauvage) dans les cellules d'eucaryotes supérieurs, même en présence de molécules activatrices de translecture (Welch *et al.,* 2007), à l'exception à ce jour de quatre gènes utilisant la translecture sur leur codon stop physiologique (Loughran *et al.,* 2014). La translecture d'un codon stop prématuré à partir d'un ARNm porteur de ce codon stop prématuré permet de conduire à la synthèse d'une protéine de taille identique à celle de la protéine sauvage et présentant au maximum un seul acide aminé muté (différent) par rapport à la protéine sauvage. En effet, l'acide aminé incorporé lors de la translecture au niveau du codon stop prématuré peut être différent de celui présent dans la protéine sauvage. Si cet acide aminé n'est pas incompatible avec la fonction de la protéine, la protéine synthétisée après translecture sera alors fonctionnelle.

Plusieurs molécules ont été identifiées pour leur capacité à induire la translecture (Benhabiles *et al.,* 2016). Il s'agit notamment de certains membres de la famille des aminoglycosides comme la gentamycine ou la généticine (aussi appelée G418) et des molécules n'appartenant pas à cette famille comme l'ataluren (aussi appelé PTC124 ou Translarna) ou l'amlexanox (Du *et al.,* 2009; Gonzalez-Hilarion *et al.,* 2012; Hermann, 2007; Keeling *et al.,* 2001; Keeling *et al.,* 2006; Welch *et al.,* 2007). Cependant, ces molécules ont une efficacité de translecture très limitée et/ou une toxicité importante. Par exemple, il n'a pas été clairement démontré chez des patients atteints de myopathie de Duchenne liée à une mutation non-sens, que l'ataluren pouvait restaurer l'expression d'un gène porteur de cette mutation non-sens (Fitzhugh and Writer, 2016). Le G418 est une des molécules activant la translecture les plus efficaces (Bidou *et al.,* 2004; Dranchak *et al.,* 2011; Sangkuhl *et al.,* 2004), mais sa toxicité ne permet pas un développement thérapeutique (Swan, 1997).

Par ailleurs, Taanman *et al.* (2003) ont montré *in vitro* à partir de fibroblastes de peau prélevés chez un patient portant une mutation non-sens non décrite du gène codant la désoxyguanosine kinase (mutation dont la déficience en désoxyguanosine kinase est responsable du syndrome de déplétion de l'ADN mitochondrial du fait d'un pool (réservoir) en désoxyguanosine monophosphate (dGMP) et en désoxyadénosine monophosphate (dAMP) restreint), que la supplémentation en dGMP ou en dAMP permet de prévenir la déplétion de l'ADN mitochondrial. La supplémentation en dGMP ou en dAMP permet donc de reconstituer le pool de désoxyribonucléides dans ces cellules porteuses d'une mutation non-sens.

Au regard du manque de molécules permettant une correction efficace des mutations non-sens, il existe un besoin d'identifier de nouvelles molécules possédant cette propriété.

Les Inventeurs ont montré que des dérivés de la purine, en particulier la 2,6-diaminopurine (désignée indifféremment DAP ou 2,6-DAP dans ce qui suit), possèdent une activité correctrice sélective sur le codon stop UGA introduit par une mutation non-sens. Les Inventeurs ont aussi montré *in vitro* que la DAP possède une activité correctrice de cette mutation non-sens UGA, significativement supérieure à celle du composé G418 et de l'ataluren.

La DAP est également connue pour son utilisation dans le traitement de la leucémie (Burchenal *et al.,* 1949) et son activité antivirale (Friend, 1951). Par ailleurs, l'utilisation de la DAP et de ses analogues est également étudiée dans le but de traiter des maladies ayant une composante inflammatoire, comme présenté dans les travaux du document WO03/0045112.

La formule suivante représente la molécule de purine avec la numérotation des positions qui sera utilisée dans la suite de la description :

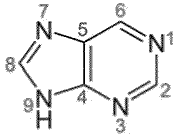

La présente invention a pour objet :
- la 2,6-diaminopurine (DAP) pour son utilisation dans le traitement d'une maladie due une mutation non-sens dans un gène conduisant à l'introduction prématurée d'un codon stop UGA ;
- en particulier, la DAP pour son utilisation pour le traitement d'une maladie choisie parmi la mucoviscidose due à ladite mutation non-sens, les dystrophies musculaires dues à ladite mutation non-sens, l'hémophilie due à ladite mutation non-sens, la béta-thalassémie due à ladite mutation non-sens, la rétinite pigmentaire due à ladite mutation non-sens, les mucopolysaccharidoses dues à ladite mutation non-sens, l'amyotrophie spinale due à ladite mutation non-sens ;
- la DAP pour son utilisation selon les points qui précèdent, caractérisée en ce qu'elle est destinée à être administrée à un sujet en combinaison avec un composé possédant une activité de translecture choisi dans le groupe constitué par la 6-amino-5-nitro-4-(α-D-ribofuranosylamino)-pyrimidine, la 6-amino-5-nitro-4-(β-D-ribofuranosylamino)-pyrimidine, l'ataluren, la gentamycine, la geneticine, la paromomycine et les dérivés de la paromomycine tels que NB30; NB54; NB74; NB84, l'amikacine, la tobramycine, la pyramicine et les dérivés de la pyramycine tels que TC001 ; TC003; TC007; TC032, la kanamycine et les dérivés de la kanamycine tels que JL022; JL023, l'amlexanox, le RTC 13, le RTC 14, le 3-(2-4E(l,l dimethyl propyl)-phenoxy-acetylamino)-benzoic acid, le 3-(2-(4-isopropyl-3-methyl-phenoxy)-acetylamino)-benzoic acid, la negamycine, la tylosine, la josamycine, la spiramycine, et le 3-(2-(4-tert-butyl-phenoxy)-acetylamino)-benzoic acid.

La 2,6-diaminopurine (DAP) possède la formule (I) :

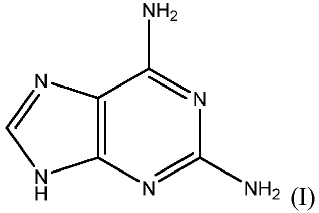

La DAP peut être sous la forme d'un sel pharmaceutiquement acceptable.

Un sel pharmaceutiquement acceptable de la DAP comprend les sels d'addition à un acide ou à une base de la DAP. Des sels d'addition à un acide appropriés sont formés à partir d'acides qui forment des sels non toxiques. Des exemples de sels d'addition à un acide incluent, mais ne sont pas limités aux sels d'acétate, de trifluoroacétate, d'adipate, d'aspartate, de benzoate, de bésylate, de bicarbonate/carbonate, de bisulphate/sulphate, de borate, de tétrafluoroborate, de camsylate, de citrate, de cyclamate, d'édisylate, d'ésylate, de formate, de fumarate, de gluceptate, de gluconate, de glucuronate, d'hexafluorophosphate, d'hibenzate, de chlorhydrate/chlorure, de bromhydrate/bromure, d'iodhydrate/iodure, d'iséthionate, de lactate, de malate, de maléate, de malonate, de mésylate, de sulfate de méthyle, de naphthylate, de 2-napsylate, de nicotinate, de nitrate, d'orotate, d'oxalate, de palmitate, de pamoate, de phosphate, de phosphate monohydrogéné, de phosphate dihydrogéné, de pyroglutamate, de saccharate, de stéarate, de succinate, de tannate, de tartrate, de tosylate, de trifluoroacétate et de xinofoate. Des sels d'addition à une base appropriés sont formés à partir de bases qui forment des sels non toxiques. Des exemples de sels d'addition à une base incluent, mais ne sont pas limités aux sels d'aluminium, d'arginine, de benzathine, de calcium, de choline, de diéthylamine, de diolamine, de glycine, de lysine, de magnésium, de méglumine, d'olamine, de potassium, de sodium, de trométhamine, de 2-(diéthylamino)éthanol, d'éthanolamine, de morpholine, 4- (2-hydroxyéthyl)morpholine et de zinc. De préférence, les sels pharmaceutiquement acceptables incluent le chlorhydrate/chlorure, le bromhydrate/bromure, le bisulfate/sulfate, le nitrate, le citrate et l'acétate.

La DAP peut être sous la forme d'un solvate.

Le terme « solvate » désigne la DAP comprenant des quantités stoechiométriques ou sous-stoechiométriques d'une ou de plus d'une molécule d'un solvant pharmaceutiquement acceptable telle que l'éthanol.

On entend par « maladie due à une mutation non-sens d'un gène conduisant à l'introduction prématurée d'un codon stop UGA », une maladie causée par la présence d'une mutation non-sens affectant un gène d'intérêt dans les cellules germinales et/ou les cellules somatiques ; la mutation non-sens étant une mutation ponctuelle d'un codon qui entraîne le changement d'un codon codant un acide aminé en un codon stop UGA qui entraîne l'arrêt de la traduction.

Plusieurs maladies dues à cette mutation non-sens ont été décrites (Keeling *et al.,* 2006; Bidou *et al.,* 2012; Lee and Dougherty, 2012 ; Kosuga *et al.,* 2016). Ces maladies peuvent affecter différents organes, tels que le foie, les intestins, le rein, le poumon, le muscle, la moelle osseuse ou le système nerveux central.

Les maladies dues à la mutation non-sens d'un gène conduisant à l'introduction prématurée d'un codon stop UGA peuvent être identifiées par des méthodes de génotypage des cellules du sujet à traiter, plus particulièrement par séquençage ou par quantification de l'expression du gène d'intérêt. Ces méthodes sont bien connues de l'homme du métier (Baharin *et al.,* 2015 ; Bladen *et al.,* 2015 ; Cangül *et al.,* 2015 ; Carmosino *et al.,* 2016 ; Csànyi *et al.,* 2016 ; Kosuga *et al.,* 2016 ; Lin *et al.,* 2016 ; Roosing *et al.,* 2016 ; Xia *et al.,* 2016 ; Zemrani *et al.,* 2016 ; Zhao *et al.,* 2016). Par exemple, la Demande Internationale WO 2012/016930 décrit de manière générale une méthode pour déterminer si une maladie est due à cette mutation non-sens.

Les maladies dues à ladite mutation non-sens incluent les maladies inflammatoires dues à ladite mutation non-sens, les maladies neurodégénératives dues à ladite mutation non-sens, les maladies auto-immunes dues à ladite mutation non-sens, les maladies cardiovasculaires dues à ladite mutation non-sens, les maladies pulmonaires dues à ladite mutation non-sens, les cancers dus à ladite mutation non-sens, l'amylose due à ladite mutation non-sens, la maladie d'Alzheimer due à ladite mutation non-sens, l'athérosclérose due à ladite mutation non-sens, le gigantisme dû à ladite mutation non-sens, le nanisme dû à ladite mutation non-sens, l'hypothyroïdie due à ladite mutation non-sens, l'hyperthyroïdie due à ladite mutation non-sens, la mucoviscidose due à ladite mutation non-sens, l'obésité due à ladite mutation non-sens, la maladie de Parkinson due à ladite mutation non-sens, la maladie de Niemann Pick due à ladite mutation non-sens, l'hypercholestérolémie familiale due à ladite mutation non-sens, la rétinite pigmentaire due à ladite mutation non-sens, le syndrome de Marfan dû à ladite mutation non-sens, les maladies lysosomales dues à ladite mutation non-sens, les dystrophies musculaires dues à ladite mutation non-sens, l'hémophilie due à ladite mutation non-sens, les céroïde-lipofuscinoses neuronales infantiles tardives dues à ladite mutation non-sens, la béta-thalassémie due à ladite mutation non-sens, le syndrome d'Ehlers Danlos dû à ladite mutation non-sens, le syndrome de Dravet dû à ladite mutation non-sens, l'achromatopsie due à ladite mutation non-sens, la rétinite pigmentaire due à ladite mutation non-sens, le syndrome d'Usher de type IC dû à ladite mutation non-sens, le syndrome d'Elhers-Danlos type musculo-contractural dû à ladite mutation non-sens, le syndrome Alagille dû à ladite mutation non-sens, le syndrome d'Alström dû à ladite mutation non-sens, un déficit en antithrombine dû à ladite mutation non-sens, le complexe de Carney dû à ladite mutation non-sens, le syndrome de Currarinon dû à ladite mutation non-sens, l'anémie de Blackfan-Diamond due à ladite mutation non-sens, la protoporphyrie érythropoïétique due à ladite mutation non-sens, la maladie de Fabry due à ladite mutation non-sens, le déficit congénital en facteur XIII dû à ladite mutation non-sens, la glycogénose de Bickel-Fanconi due à ladite mutation non-sens, la triméthylaminurie due à ladite mutation non-sens, la maladie de Gaucher due à ladite mutation non-sens, la maladie de Rendu-Osler due à ladite mutation non-sens, l'homocystinurie due à ladite mutation non-sens, le syndrome de Joubert dû à ladite mutation non-sens, la maladie de Krabbe due à ladite mutation non-sens, l'acidurie L-2-HG due à ladite mutation non-sens, l'acidémie méthylmalonique due à ladite mutation non-sens, le syndrome de Peters-plus dû à ladite mutation non-sens, le syndrome de Townes-Brocks dû à ladite mutation non-sens, la maladie de von Willebrand due à ladite mutation non-sens, le syndrome de Wiskott-Aldrich dû à ladite mutation non-sens, le syndrome de Kabuki dû à ladite mutation non-sens, la maladie de Dorfman-Chanarin due à ladite mutation non-sens, la maladie des yeux de poisson due à un déficit partiel en lécithine-cholestérol-acyl-transférase due à ladite mutation non-sens, les mucopolysaccharidoses dues à ladite mutation non-sens, le déficit en coenzyme Q10 dû à ladite mutation non-sens, le syndrome de Zellweger dû à ladite mutation non-sens, le cancer colorectal dû à ladite mutation non-sens, l'entéropathie congénitale due à un déficit de l'entéropeptidase due à ladite mutation non-sens, le syndrome de Peutz-Jeghers dû à ladite mutation non-sens, le syndrome de Jervell et Lange Nielsen dû à ladite mutation non-sens, le syndrome de Lynch dû à ladite mutation non-sens, la maladie des inclusions microvillositaires due à ladite mutation non-sens, la xanthinurie due à ladite mutation non-sens, l'acidose due à ladite mutation non-sens, le syndrome d'Alport dû à ladite mutation non-sens, le syndrome de Bardet Biedl dû à ladite mutation non-sens, le syndrome de Birt-Hogg-Dubé dû à ladite mutation non-sens, la maladie de Dent due à ladite mutation non-sens, le syndrome de Gitelman dû à ladite mutation non-sens, le syndrome de léiomyomatose héréditaire-cancer du rein dû à ladite mutation non-sens, la maladie de Minkowski-Chauffard due à ladite mutation non-sens, l'amaurose congénitale de Leber due à ladite mutation non-sens, l'intolérance aux protéine dibasiques avec lysinurie due à ladite mutation non-sens, la néphronophthise due à ladite mutation non-sens, la polykystose rénale récessive due à ladite mutation non-sens, le pseudohypoaldostéronisme dû à ladite mutation non-sens, l'hypoplasie et la dysplasie rénales dues à ladite mutation non-sens, le carcinome à cellule claire du rein dû à ladite mutation non-sens, le carcinome papillaire du rein de type 2 dû à ladite mutation non-sens, le syndrome d'Ochoa dû à ladite mutation non-sens, la maladie de von Hippel-Lindau due à ladite mutation non-sens, la tumeur de Wilms due à ladite mutation non-sens, le rachitisme hypophosphatémique lié à l'X dû à ladite mutation non-sens, la néphropathie hyperuricémique familiale juvénile due à ladite mutation non-sens, la sclérose tubéreuse de Bourneville due à ladite mutation non-sens, le syndrome néphrotique finlandais dû à ladite mutation non-sens, le syndrome néphrotique idiopathique cortico-résistant, le syndrome de Pierson dû à ladite mutation non-sens, le syndrome de Denys-Drash dû à ladite mutation non-sens, le syndrome de Schimke dû à ladite mutation non-sens, la résistance au glucocorticoïde primaire due à ladite mutation non-sens, le rachitisme vitamino-résistant hypophosphatémique dû à ladite mutation non-sens, l'hyperoxalurie primitive de type 1 due à ladite mutation non-sens, le pseudohypoaldostéronisme type 1 (PHA1) dû à ladite mutation non-sens, l'acidose tubulaire rénale type 2 due à ladite mutation non-sens, la maladie de Bassen-Kornzweig due à ladite mutation non-sens, le syndrome d'Alpers Huttenlocher dû à ladite mutation non-sens, le déficit en carbamoyl-phosphate synthase I (CPS1) dû à ladite mutation non-sens, la maladie de surcharge en *esters* de cholestérol due à ladite mutation non-sens, le déficit en citrine dû à ladite mutation non-sens, le syndrome de Dubin-Johnson dû à ladite mutation non-sens, la déficience en facteur V due à ladite mutation non-sens, la glycogénose due à ladite mutation non-sens, l'hémophilie due à un déficit en facteur VIII ou IX due à ladite mutation non-sens, le carcinome hépatocellulaire dû à ladite mutation non-sens, la porphyrie hépatoérythropoïétique due à ladite mutation non-sens, la paraplégie spastique familiale due à ladite mutation non-sens, l'hypo-betalipoprotéinémie due à ladite mutation non-sens, le déficit constitutionnel en facteur XI dû à ladite mutation non-sens, le diabète de type adulte chez le jeune dû à ladite mutation non-sens, l'anémie hypochrome microcytaire due à ladite mutation non-sens, le syndrome de déplétion de l'ADN mitochondrial dû à ladite mutation non-sens, la phénylcétonurie due à ladite mutation non-sens, la polykystose hépatique due à ladite mutation non-sens, la porphyrie cutanée tardive due à ladite mutation non-sens, la cholestase intrahépatique progressive familiale due à ladite mutation non-sens, la maladie de Wilson due à ladite mutation non-sens, l'hypercholestérolémie autosomale dominante due à ladite mutation non-sens, la déficience en facteur XII due à ladite mutation non-sens, la déficience en facteur X due à ladite mutation non-sens, l'hypofibrinogénémie due à ladite mutation non-sens, l'afibrinogénémie due à ladite mutation non-sens, la déficience en facteur VII due à ladite mutation non-sens, l'agammaglobulinémie due à ladite mutation non-sens, la thrombocytopénie amégakaryocytaire due à ladite mutation non-sens, l'anémie dysérythropoïétique congénitale type 2 due à ladite mutation non-sens, les dystrophies musculaires de Duchenne (DMD) et de Becker (BMD) dues à ladite mutation non-sens, les myopathies centronucléaires dues à ladite mutation non-sens, les dystrophies musculaires des ceintures dues à ladite mutation non-sens, la myopathie de Miyoshi due à ladite mutation non-sens, la dystrophie musculaire congénitale de type Ullrich due à ladite mutation non-sens, l'amyotrophie spinale due à ladite mutation non-sens, l'épidermolyse bulleuse dystrophique due à ladite mutation non-sens, la maladie de Hailey-Hailey due à ladite mutation non-sens, l'épidermolyse bulleuse jonctionnelle type Herlitz due à ladite mutation non-sens, le syndrome de Netherton dû à ladite mutation non-sens, le syndrome de Hurler dû à ladite mutation non-sens, la LINCL (*Late Infantile Neuronal Ceroid Lipofuscinosis*) due à ladite mutation non-sens, de préférence le cancer du poumon et la mucoviscidose due à ladite mutation non-sens.

Selon un mode de réalisation particulier, la maladie due à ladite mutation non-sens est choisie parmi la mucoviscidose due à ladite mutation non-sens, les dystrophies musculaires dues à ladite mutation non-sens, l'hémophilie due à ladite mutation non-sens, la béta-thalassémie due à ladite mutation non-sens, la rétinite pigmentaire due à ladite mutation non-sens, les mucopolysaccharidoses dues à ladite mutation non-sens, l'amyotrophie spinale due à ladite mutation non-sens. WO 2016/087665 propose notamment le traitement d'une forme très particulière de la mucoviscidose avec divers composés dérivés de purine ; cette forme de mucoviscidose n'est pas induite par une mutation non-sens mais par la délétion d'une phénylalanine en position 508 (F508del) de la protéine CFTR.

En particulier, la maladie due à ladite mutation non-sens peut être la mucoviscidose due à la mutation non-sens d'un gène conduisant à l'introduction prématurée d'un codon stop UGA dans le gène CFTR. La mucoviscidose due à la mutation non-sens d'un gène conduisant à l'introduction prématurée d'un codon stop UGA dans le gène CFTR peut conduire par exemple, de manière non-limitative, aux mutations suivantes : G27X, W57X, R75X, W202X, W216X, C225X, L320X, W401X, S434X, S466X, S489X, G542X, R553X, G673X, R709X, L732X, G745X, R764X, R785X, R792X, C832X, W846X, R851X, W882X, G1003X, W1063X, W1089X, W1098X, R1102X, R1128X ; R1158X, R1162X, S1196X, W1204X, S1206X ou W1282X.

La présente invention a aussi pour objet :
- Une composition pharmaceutique comprenant la DAP et un excipient pharmaceutiquement acceptable pour son utilisation (destinés à être utilisés) dans le traitement d'une maladie due à une mutation non-sens dans un gène conduisant à l'introduction prématurée d'un codon stop UGA, telle que définie ci-dessus.- ladite composition pharmaceutique pour son utilisation est caractérisée en ce que ladite maladie est choisie parmi la mucoviscidose due à ladite mutation non-sens, les dystrophies musculaires dues à ladite mutation non-sens, l'hémophilie due à ladite mutation non-sens, la béta-thalassémie due à ladite mutation non-sens, la rétinite pigmentaire due à ladite mutation non-sens, les mucopolysaccharidoses dues à ladite mutation non-sens, l'amyotrophie spinale due à ladite mutation non-sens.
- ladite composition pharmaceutique pour son utilisation est caractérisée en ce qu'elle est destinée à être administrée à un sujet en combinaison avec un composé possédant une activité de translecture choisi dans le groupe constitué par la 6-amino-5-nitro-4-(α-D-ribofuranosylamino)-pyrimidine, la 6-amino-5-nitro-4-(β-D-riboiuranosylamino)-pyrimidine, l'ataluren, la gentamycine, la geneticine, la paromomycine et les dérivés de la paromomycine tels que B30; NB54; NB74; NB84, l'amikacine, la tobramycine, la pyramicine et les dérivés de la pyramycine tels que TCOOl ; TC003; TC007; TC032, la kanamycine et les dérivés de la kanamycine tels que JL022; JL023, l'amlexanox, le RTC 13, le RTC 14, le 3-(2-4E(l,l dimethyl propyl)-phenoxy-acetylamino)-benzoic acid, le 3-(2-(4-isopropyl-3-methyl-phenoxy)-acetylamino)-benzoic acid, la negamycine, la tylosine, la josamycine, la spiramycine, et le 3-(2-(4-tert-butyl-phenoxy)-acetylamino)-benzoic acid.

On entend par « excipient » une substance qui porte la DAP selon l'invention dans une composition lui conférant par exemple des propriétés de stabilité, de forme (par exemple liquide, solide, gélule), de goût, de dissolution (par exemple dissolution ciblée dans l'estomac ou le tube digestif) et de couleur.

Un « excipient pharmaceutiquement acceptable » concerne un excipient qui ne produit pas de réaction défavorable, allergique ou indésirable lorsqu'il est administré à un sujet. Cela inclut tous les solvants, les milieux de dispersion, les enrobages, les agents antibactériens et antifongiques, les agents isotoniques, les agents à absorption retardée et autres substances similaires. Pour une administration chez un être humain, les préparations doivent répondre aux critères de stérilité, de pyrogénicité, et aux normes de sécurité et de pureté générales requises par les offices régulateurs. L'excipient peut par exemple être de l'eau.

Les termes « traitement » ou « traiter » concernent à la fois le traitement thérapeutique et les mesures prophylactiques ou préventives, pour lesquels l'objet est d'empêcher ou de ralentir la progression de la maladie due à une mutation non-sens d'un gène conduisant à l'introduction prématurée d'un codon stop UGA. Les sujets qui ont besoin d'un traitement incluent ceux qui ont déjà une maladie due à une mutation non-sens d'un gène conduisant à l'introduction prématurée d'un codon stop UGA, ceux prédisposés à une maladie due à une mutation non-sens d'un gène conduisant à l'introduction prématurée d'un codon stop UGA et ceux chez qui une maladie due à une mutation non-sens d'un gène conduisant à l'introduction prématurée d'un codon stop UGA doit être prévenue.

Un sujet est traité avec succès pour une maladie due à une mutation non-sens d'un gène conduisant à l'introduction prématurée d'un codon stop UGA si, après avoir reçu une quantité thérapeutiquement efficace de DAP ou d'une composition pharmaceutique selon l'invention, le sujet montre une réduction observable ou mesurable, ou l'absence, de l'un au moins des points suivants : réduction du nombre de cellules pathogéniques, réduction du pourcentage de cellules pathogéniques par rapport aux cellules totales, et/ou de l'un ou de plusieurs des symptômes associés à la maladie due à une mutation non-sens d'un gène conduisant à l'introduction prématurée d'un codon stop UGA ou une amélioration de la qualité de vie. Les paramètres d'évaluation ci-dessus sont facilement mesurables par les procédures de routine familières à un médecin.

Avantageusement, les patients sont présélectionnés comme présentant dans un gène d'intérêt ladite mutation non-sens.

Le terme « sujet » concerne un mammifère, de préférence un humain. Dans un mode de réalisation préféré, le sujet peut être un "patient", *i.e.* un animal à sang chaud, de préférence un humain, en attente de recevoir ou recevant des soins médicaux, qui a fait l'objet d'une procédure médicale, ou qui est suivi pour le développement d'une maladie génétique liée à une mutation non-sens d'un gène conduisant à l'introduction prématurée d'un codon stop UGA.

Une « quantité thérapeutiquement efficace » concerne la quantité de DAP ou de composition pharmaceutique nécessaire et suffisante pour, sans causer d'effets secondaires significatifs et défavorables pour le sujet, diminuer ou stopper la progression, ou l'aggravation de l'un ou de plusieurs des symptômes de la maladie due à une mutation non-sens d'un gène conduisant à l'introduction prématurée d'un codon stop UGA, pour soulager les symptômes de la maladie due à une mutation non-sens d'un gène conduisant à l'introduction prématurée d'un codon stop UGA, et/ou pour guérir la maladie due à une mutation non-sens d'un gène conduisant à l'introduction prématurée d'un codon stop UGA.

Les modes et voies d'administration de la DAP ou d'une composition pharmaceutique tels que définis ci-dessus peuvent être adaptés par l'homme du métier en fonction du sujet et du dérivé de la purine utilisé. A titre d'exemple, la DAP selon l'invention peut être formulé pour une administration par voie orale ou nasale, ou par injection par voie intraveineuse, intramusculaire ou sous-cutanée, de préférence par voie orale.

La détermination de la dose à laquelle ladite DAP est utilisée peut s'effectuer par des techniques connues de l'homme du métier, par exemple lors d'essais cliniques. Cette dose dépendra de divers facteurs comprenant notamment l'activité du dérivé de la purine selon l'invention, du mode d'administration, de la durée de l'administration, de la durée du traitement, d'autres médicaments ou composés utilisés en combinaison avec ledit dérivé de la purine selon l'invention, de l'âge, du sexe, du poids, de l'état de santé générale et de l'histoire médicale antérieure du sujet qui est traité.

Selon un mode de réalisation avantageux, la DAP est administré à un sujet en combinaison avec un composé possédant une activité de translecture choisi dans le groupe constitué par l'anomère alpha de la clitocine (6-amino-5-nitro-4-(α-D-ribofuranosylamino)-pyrimidine), l'anomère beta de la clitocine (6-amino-5-nitro-4-(β-D-ribofuranosylamino)-pyrimidine), l'ataluren, la gentamycine, la geneticine, la paromomycine et les dérivés de la paromomycine tels que NB30; NB54; NB74; NB84, l'amikacine, la tobramycine, la pyramicine et les dérivés de la pyramycine tels que TC001; TC003; TC007; TC032, la kanamycine et les dérivés de la kanamycine tels que JL022; JL023, l'amlexanox, le RTC 13 (Lavin, 2013), le RTC 14 (Lavin, 2013), le 3-(2-4E(1,1 dimethyl propyl)-phenoxy-acetylamino)-benzoic acid, le 3-(2-(4-isopropyl-3-methyl-phenoxy)-acetylamino)-benzoic acid, la negamycine, la tylosine, la josamycine, la spiramycine, et le 3-(2-(4-tert-butyl-phenoxy)-acetylamino)-benzoic acid ; de préférence le composé possédant une activité de translecture est choisi dans le groupe constitué par l'anomère alpha de la clitocine (6-amino-5-nitro-4-(α-D-ribofuranosylamino)-pyrimidine), l'anomère beta de la clitocine (6-amino-5-nitro-4-(β-D-ribofuranosylamino)-pyrimidine) et l'ataluren.

La présente invention a aussi pour objet :
- Une composition comprenant la DAP et un composé possédant une activité de translecture choisi dans le groupe constitué par l'anomère alpha de la clitocine (6-amino-5-nitro-4-(α-D-ribofuranosylamino)-pyrimidine), l'anomère beta de la clitocine (6-amino-5-nitro-4-(β-D-ribofuranosylamino)-pyrimidine), l'ataluren, la gentamycine, la geneticine, la paromomycine et les dérivés de la paromomycine tels que NB30; NB54; NB74; NB84, l'amikacine, la tobramycine, la pyramicine et les dérivés de la pyramycine tels que TC001; TC003; TC007; TC032, la kanamycine et les dérivés de la kanamycine tels que JL022; JL023, l'amlexanox, le RTC 13 (Lavin, 2013), le RTC 14 (Lavin, 2013), le 3-(2-4E(1,1 dimethyl propyl)-phenoxy-acetylamino)-benzoic acid, le 3-(2-(4-isopropyl-3-methyl-phenoxy)-acetylamino)-benzoic acid, la negamycine, la tylosine, la josamycine, la spiramycine, et le 3-(2-(4-tert-butyl-phenoxy)-acetylamino)-benzoic acid pour leur utilisation simultanée, séparée ou séquentielle pour le traitement d'une maladie due à une mutation non-sens d'un gène conduisant à l'introduction prématurée d'un codon stop UGA telle que définie ci-dessus ; de préférence la composition comprend la DAP et un composé possédant une activité de translecture choisi dans le groupe constitué par l'anomère alpha de la clitocine (6-amino-5-nitro-4-(α-D-ribofuranosylamino)-pyrimidine), l'anomère beta de la clitocine (6-amino-5-nitro-4-(β-D-ribofuranosylamino)-pyrimidine) et l'ataluren, pour leur utilisation simultanée, séparée ou séquentielle pour le traitement d'une maladie due à une mutation non-sens d'un gène conduisant à l'introduction prématurée d'un codon stop UGA telle que définie ci-dessus.

Outre les dispositions qui précèdent, l'invention comprend encore d'autres dispositions non-limitatives, qui ressortiront des exemples expérimentaux ci-dessous, ainsi que des figures annexées :

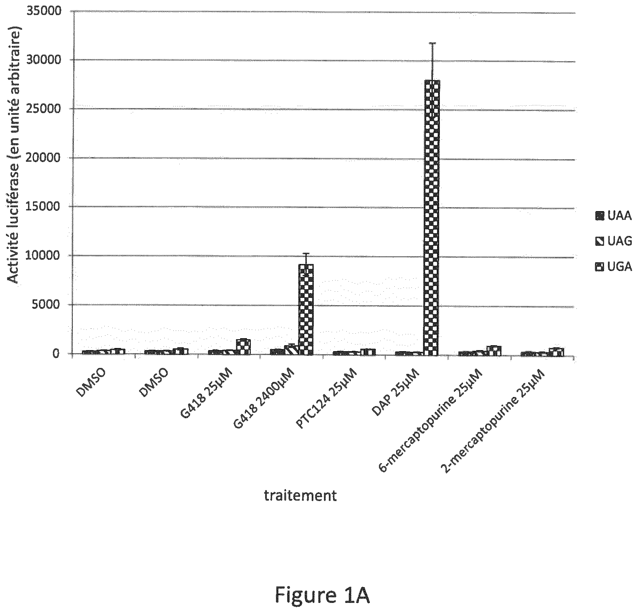

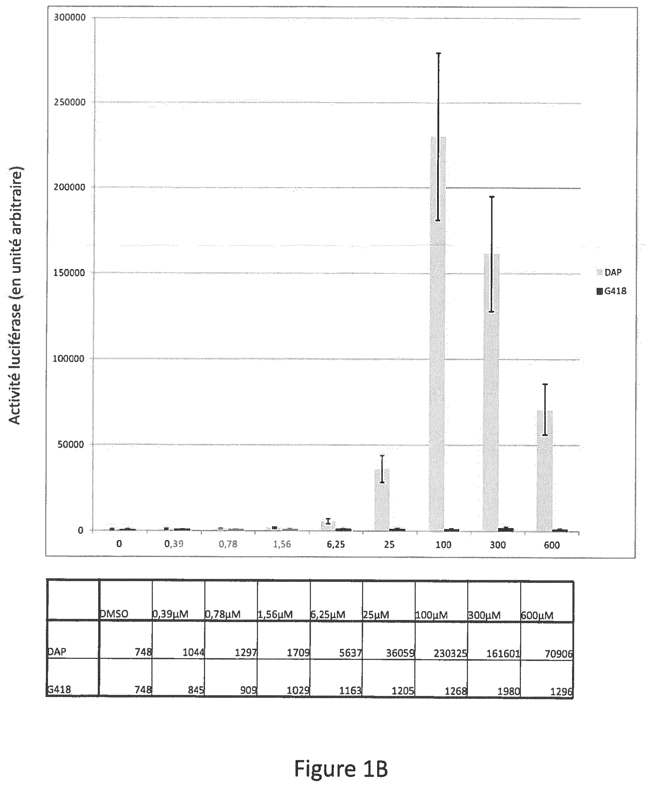

**Figure 1** : Identification de la DAP comme correcteur de mutation non-sens UGA par criblage. **A.** Mesure de l'activité luciférase dans les cellules humaines HeLa exprimant un gène luciférase porteur d'un codon stop prématuré UAA, UAG ou UGA cultivées en présence de G418 (25 µM, 2400 µM), PTC124 (ataluren) (25 µM), DAP (25 µM), 6-mercaptopurine (25 µM), 2-mercaptopurine (25 µM) ou de DMSO comme contrôle négatif. La DAP à 25 µM montre une correction de la mutation non-sens UGA de plus de trois fois plus efficace que le G418 à 2400 µM et dix-huit fois plus efficace que le G418 à 25 µM dans ces conditions expérimentales. Dans ce modèle, l'ataluren est inefficace à 25 µM. Des dérivés de la DAP (le 2-mercaptopurine ou le 6-mercaptopurine) ne possèdent pas d'activité correctrice de mutations non-sens. **B.** Mesure de l'activité luciférase à partir de cellules HeLa exprimant un gène luciférase porteur d'une mutation non sens UGA en présence de concentrations croissantes de DAP ou de G418 (0,39 µM à 600 µM), ou de DMSO (contrôle négatif). Les valeurs indiquées représentent une moyenne de deux courbes de dose/réponse pour chaque traitement et les barres d'erreur correspondent au calcul de l'écart-type.

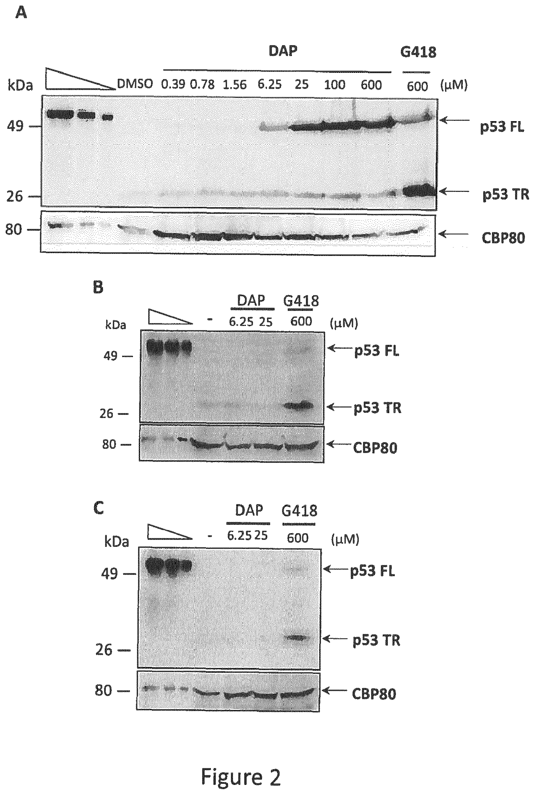

**Figure 2** **:** Analyse par *Western blot* de la réexpression du gène *TP53* porteur de mutation non-sens. **A.** Expérience de dose/réponse de la DAP et comparaison avec le G418. Les protéines des cellules Calu-6 (porteuses d'une mutation non-sens UGA au codon 196 dans le gène *TP53*) cultivées en présence de concentrations croissantes de DAP (0,39 µM à 600 µM), de G418 (600 µM) ou de DMSO comme contrôle négatif sont purifiées et analysées par *Western blot* pour détecter la protéine p53. Lorsque ces cellules sont incubées en présence de DMSO, l'expression du gène *TP53* n'est pas détectable par *Western blot* du fait de la présence de la mutation non-sens. En présence de DAP, la protéine p53 de taille sauvage (p53 FL: p53 full length) est détectée à partir de 6,25 µM et atteind une production maximale à 25 µM. L'utilisation de G418 à 600 µM permet la synthèse d'une quantité de protéine p53 identique à celle observée avec la DAP à 6,25 µM et une quantité plus importante de protéine p53 tronquée (p53 TR) dont la synthèse s'arrête à la mutation non-sens. **B.** Analyse de l'effet de la DAP ou du G418 sur des cellules Caco-2 porteuses d'une mutation UAG au niveau du codon 204 du gène *TP53.* La DAP ne permet pas de réexprimer la protéine p53 contrairement au G418. **C.** Analyse de l'effet de la DAP ou du G418 sur des cellules Caov-3 porteuses d'une mutation non-sens UAA au niveau du codon 136 du gène *TP53.* La DAP ne permet pas de réexprimer la protéine p53 contrairement au G418 qui permet une très faible réexpression. Dans toutes les expériences, la protéine CBP80 est utilisée comme contrôle de charge et les 3 pistes les plus à gauche représentent des dilutions en série d'un extrait de cellule HeLa exprimant la protéine p53 sauvage.

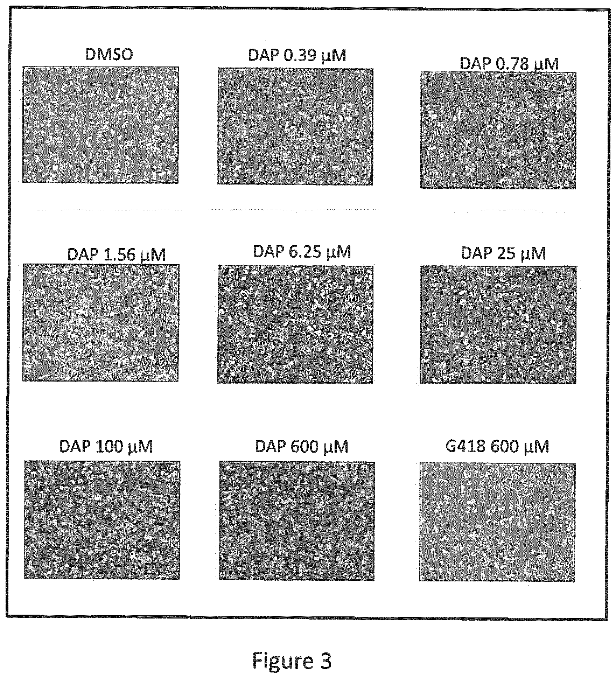

**Figure 3** **:** Effet de la DAP sur le phénotype de cellules en culture. Photographie en lumière blanche des cellules Calu-6 cultivées en présence de concentrations croissantes de DAP (0,39 µM à 600 µM), de G418 (600 µM) ou de DMSO comme contrôle négatif. L'acquisition a été faite avec un microscope ZEISS Axiovert 40C au grossissement 5x.

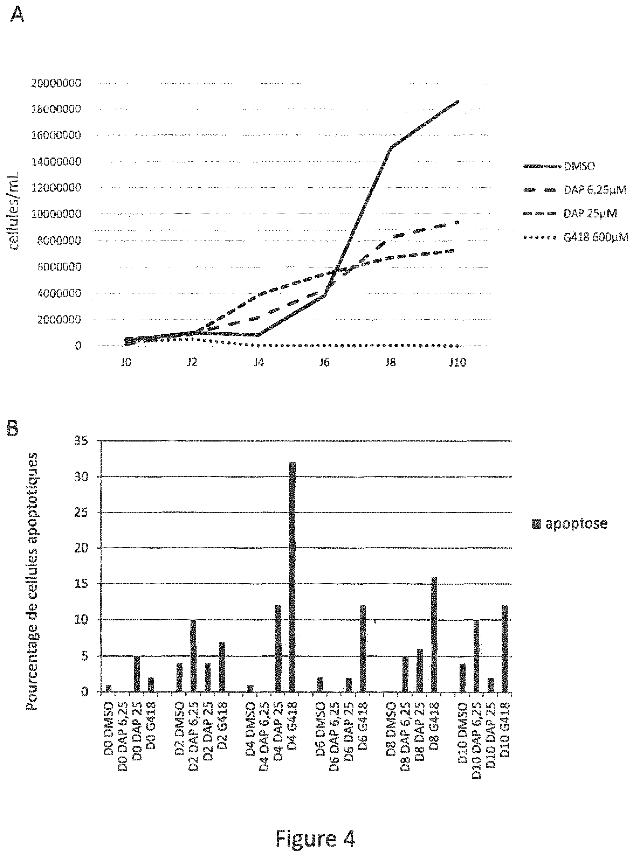

**Figure 4** **:** Mesure de la toxicité cellulaire de la DAP. Les cellules Calu-6 sont cultivées en présence de DAP (6,25 µM, 25 µM), G418 (600 µM) et de DMSO (contrôle négatif) pendant 10 jours. Les cellules sont initialement ensemencées à 50000 cellules par puits (J0). Un comptage cellulaire (**A**) ainsi que la mesure du taux d'apoptose (**B**) sont effectués dans chacune des conditions à J4, J6, J8 et J10.

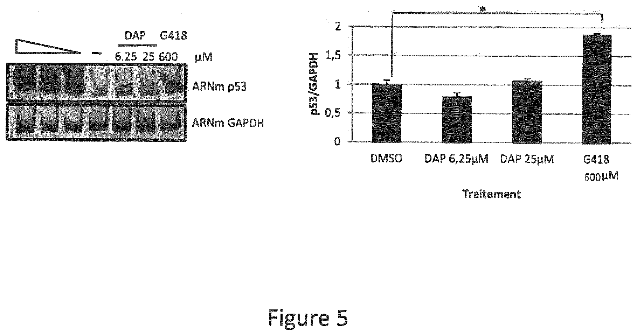

**Figure 5** **:** La DAP n'inhibe pas le NMD. Les cellules Calu-6 sont cultivées pendant 24 heures en présence de DAP (6,25 µM ou 25 µM), G418 (600 µM), DMSO (contrôle négatif). L'ARN des cellules Calu-6 est extrait et soumis à une RT-PCR en présence d'un nucléotide radiomarqué pour quantifier les niveaux d'ARNm p53 et GAPDH (comme témoin de charge) amplifiés. Les 3 pistes les plus à gauche représentent une série de dilutions de RT issue d'ARNm provenant de cellules HeLa. Une représentation sous forme d'histogramme est montrée à droite. *: p<0,05.

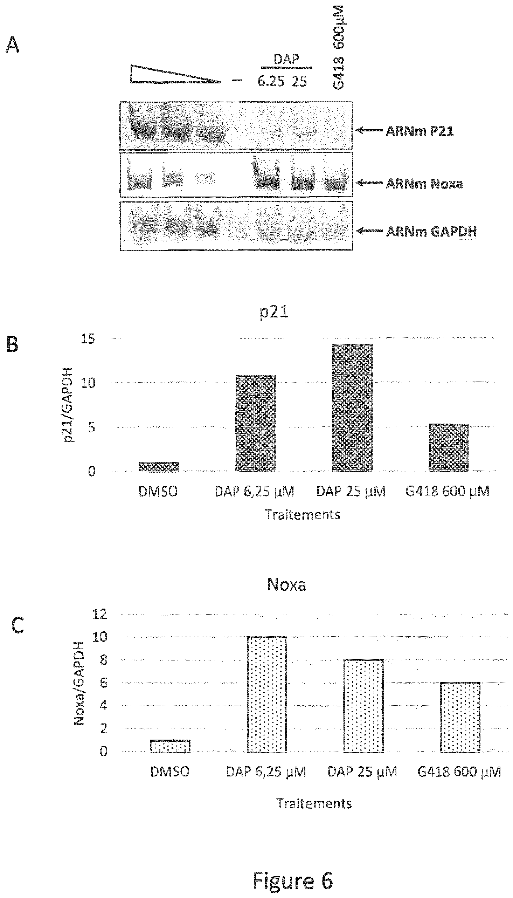

**Figure 6****.** Mesure de l'expression de deux gènes cibles de p53, p21 et Noxa, dans les cellules Calu-6 après culture en présence de DAP (6,25 µM ou 25 µM), G418 (600 µM) ou DMSO (contrôle négatif). Les ARNs des cellules Calu-6 après culture sont purifiés, rétro-transcrits et amplifiés par PCR, puis déposés sur gel pour évaluer leurs quantités par rapport au GAPDH (**A**). Les graphiques représentent le rapport du niveau d'expression d'ARNm p21 (**B**), Noxa (**C**) à celui du GAPDH dans les différentes conditions de culture. En présence de DAP ou de G418, les niveaux des ARNm issus de gènes cibles de p53 augmentent, reflétant la synthèse d'une protéine p53 fonctionnelle dans ces cellules contrairement au traitement de ces cellules par du DMSO.

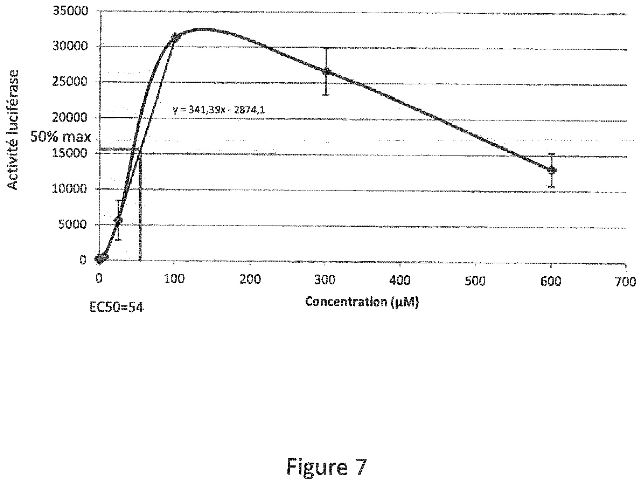

**Figure 7** **:** Caractérisation de la concentration optimale d'utilisation de la DAP. Mesure de l'activité luciférase dans les cellules Hela humaines exprimant un gène luciférase porteur d'un codon stop prématuré UGA cultivées en présence de concentrations croissantes de DAP (0-600 µM).

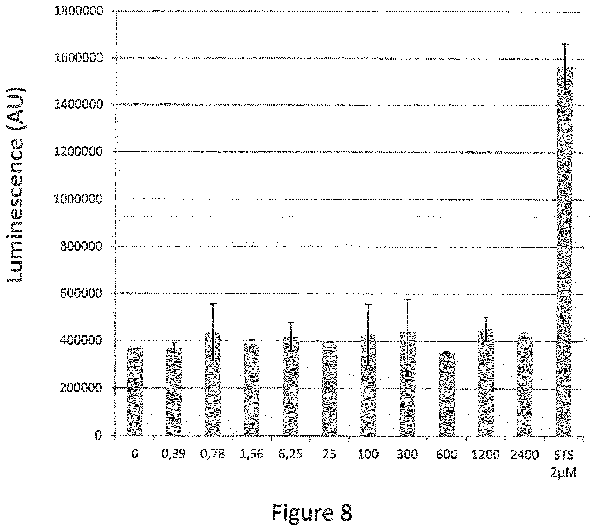

**Figure 8** **:** Mesure de la cytotoxicité des cellules Hela en présence de DAP à différentes concentrations par le kit ToxiLight^{™} (Lonza).

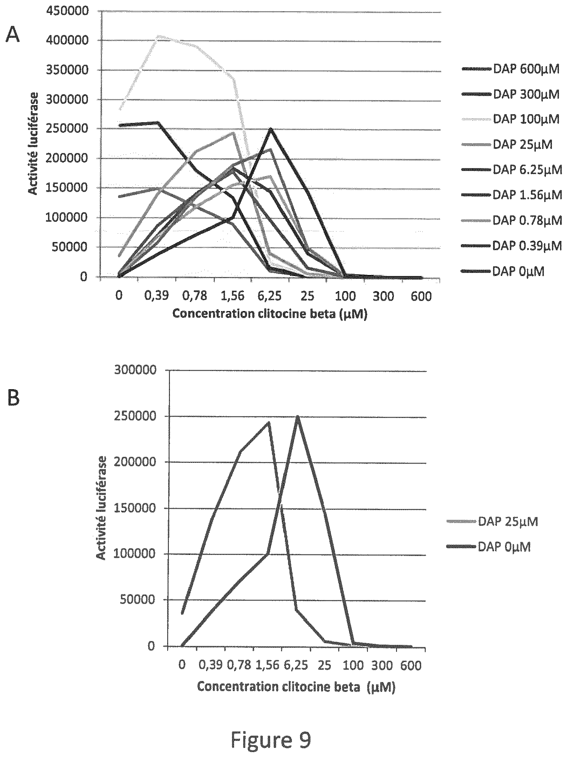

**Figure 9** : Effet synergique entre la DAP et la clitocine. A. Mesure de l'activité luciférase des cellules Hela humaines exprimant un gène luciférase porteur d'un codon stop prématuré UGA cultivées en présence de concentrations croissantes de DAP (courbes) et de clitocine (abscisse). B. Mesures de l'activité luciférase des cellules Hela humaines exprimant un gène luciférase porteur d'un codon stop prématuré UGA en présence de DAP à 0 µM et 25 µM extraites de A illustrant l'effet synergique de la DAP et la clitocine.

### EXEMPLES

### 1. Identification de la 2,6-diaminopurine (DAP) comme correcteur de mutation non-sens UGA

Afin d'identifier des molécules capables de corriger efficacement les mutations non-sens dans des cellules humaines, le gène codant la *"firefly luciférase"* (luciférase du ver luisant) a été modifié en introduisant un intron entre les codons 448 et 449 de la phase ouverte de lecture rendant ainsi l'expression de la luciférase dépendante de l'épissage, ce qui représente la situation de plus de 90% des gènes humains. Un codon stop prématuré (TAA, TAG ou TGA) a ensuite été introduit à la place du codon 109, position conduisant à activer le NMD (« nonsense-mediated mRNA decay ») sur cet ARNm. Il a donc été recréé un contexte d'expression d'un gène épissé soumis au NMD comme cela est le cas dans la grande majorité des gènes de cellules humaines lorsqu'ils sont porteurs d'une mutation non-sens. La position du codon stop prématuré est telle que si la synthèse protéique s'arrête au codon stop prématuré, la protéine luciférase tronquée synthétisée n'est pas fonctionnelle. Une activité luciférase sera par conséquent mesurée supérieure au bruit de fond lorsque l'ARNm luciférase subira de la translecture au niveau de son codon stop prématuré et l'intensité de l'activité est directement reliée à la quantité de luciférase fonctionnelle synthétisée, donc translue.

Pour cela, les cellules HeLa sont transfectées avec une construction luciférase portant une des 3 mutations non-sens (UAA, UAG et UGA) en utilisant de la lipofectamine 3000 selon le protocole du fabricant (Lifetechnologies). Le jour suivant, les cellules sont réparties en plaque 96 puits et les molécules chimiques à tester sont ajoutées immédiatement avant une incubation de 24 heures. La DAP, le 6-mercaptopurine, le 2-mercaptopurine, l'ataluren, (PTC124) ont été ajoutés à une concentration de 25 µM. Le G418 a été ajouté à une concentration de 25 µM et 2400 µM. Le DMSO représente le contrôle négatif. Le substrat SteadyLite plus (Perkin Elmer) a ensuite été ajouté au milieu de culture et les plaques ont été lues au luminomètre Tristar (Berthold). Chaque puits est lu sur 10 secondes et la plaque est lue deux fois.

En utilisant ces constructions, la 2,6 diaminopurine (DAP) a été identifiée comme une molécule capable de corriger les codons stop UGA bien plus efficacement que le G418 ou l'ataluren (PTC124) qui ne montre aucune activité de correction à cette concentration et sur ce modèle d'étude. Par contre, la DAP n'a aucun effet sur les codons UAG ou UAA faisant de cette molécule un correcteur exclusif de mutations UGA (Figure 1A). Deux dérivés de la DAP ont aussi été testés (la 2-mercaptopurine et la 6-mercaptopurine) mais ne montrent aucune activité correctrice de mutation non-sens.

Une gamme de dose-réponse est présentée en Figure 1B pour la DAP et le G418 à une concentration allant de 0,39 µM à 600 µM afin de montrer la différence d'efficacité des deux molécules pour la correction d'une mutation non-sens UGA. Le DMSO représente le contrôle négatif. C'est à partir de 6,25 µM que les deux molécules commencent significativement à corriger la mutation non-sens. Dès cette concentration, la DAP induit une correction cinq fois plus importante que le G418 sur ce modèle. On peut noter que la DAP atteint son maximum d'efficacité de correction à 100 µM contrairement au G418 qui ne l'atteint pas même à 600 µM dans ces conditions expérimentales. A 100 µM, la correction de la mutation non-sens UGA par la DAP est plus de 180 fois supérieure à celle obtenue par le G418 (voir Figure 4).

### 2. Identification de la DAP comme un correcteur de mutation non-sens UGA par translecture.

Dans le but de valider les résultats du crible ci-dessus, la correction de mutations non-sens a été mesurée pour des gènes endogènes porteurs de mutations non-sens dans une lignée cellulaire de cancer de poumons Calu-6 (ATCC, USA) comprenant une mutation UGA dans le codon 196 du gène *TP53* (Figure 2A), une lignée cellulaire Caco-2 porteuse d'une mutation UAG au niveau du codon 204 (Figure 2B) et une lignée cellulaire Caov-3 porteuses d'une mutation non-sens UAA au niveau du codon 136 (Figure 2C).

Ces cellules ont été cultivées en présence de concentrations croissantes de DAP (0,39 µM ; 0,78 µM ; 1,56 µM ; 6,25 µM ; 25 µM ; 100 µM ; 600 µM), G418 (600 µM) ou de DMSO comme contrôle négatif pendant 20 heures. La présence de la forme tronquée (p53 TR) et complète (FL) de la protéine p53 est révélée par *Western blot.* Les protéines sont extraites des cellules dans un tampon de lyse, puis analysées par gel SDS-PAGE à 10% comme précédemment décrit dans Gonzalez-Hilarion *et al.* (2012). Brièvement, après migration, les protéines ont été transférées sur une membrane de nitrocellulose et mises en présence d'un anticorps anti-p53 (D01; Santa-Cruz), puis d'un anticorps secondaire anti-souris (Jackson Immuno Research). Les protéines ont été finalement révélées en utilisant le substrat SuperSignal West Femto Maximum Sensitivity.

Les résultats (Figure 2) montrent que la DAP est capable de restaurer l'expression de gènes porteurs de mutation non-sens de type UGA, validant ainsi le résultat du crible.

La concentration minimale permettant cette réexpression sur ces lignées est de 6,25 µM pour laquelle la quantité de protéine synthétisée est semblable à la quantité obtenue avec du G418 à la concentration de 600 µM, qui est une concentration généralement utilisée pour montrer de la translecture par la G418 (Bidou *et al.,* 2004). A 25 µM de DAP, une synthèse de protéine translue plus importante qu'avec le G418 est atteinte.

### 3. La DAP ne montre pas ou peu de toxicité à des concentrations permettant la correction de mutations UGA

Les cellules Calu-6 traitées avec de la DAP ou du G418 aux différentes concentrations comme précédemment décrit sont observées au microscope en lumière blanche (grossissement 5x) afin de visualiser des changements phénotypiques des cellules.

Les résultats sont montrés à la Figure 3. Jusqu'à 25 µM de DAP, le phénotype des cellules est semblable à celui des cellules exposées au DMSO. A partir de 600 µM de DAP, on observe plus de cellules mourantes (aspect rond) représentant une situation très similaire à celle observée en incubant les cellules avec le G418 à la concentration de 600 µM. Ce résultat suggère que dans ces conditions expérimentales, la DAP n'est pas ou peu toxique à la concentration de 6,25 µM ou 25 µM, concentrations suffisantes pour obtenir la translecture des codons stop prématurés UGA (Figure 2).

### 4. Croissance cellulaire et mesure de l'apoptose

Afin de poursuivre l'étude de la toxicité de la DAP, la croissance de cellules Calu-6 a été suivie en l'absence ou en présence de DAP ou de G418.

50000 cellules Calu-6 ont été ensemencées dans des plaques 6 puits en présence de DAP (6,25 µM ou 25 µM), G418 (600 µM) ou de DMSO comme contrôle négatif. Toutes les 48 heures, pour chaque condition de traitement, les cellules ont été récupérées après digestion à la trypsine et comptées au cytomètre TALI (Lifetechnologies) puis analysées pour l'apoptose comme décrit précédemment dans Jia *et al.,* 2015. Brièvement, les cellules sont décrochées de leur support en utilisant de la trypsine puis centrifugées 5 minutes à 200g. Le culot cellulaire est alors lysé puis traité pour détecter les cellules apoptotiques en utilisant le kit Tali^{®} Apoptosis Kit - Annexin V Alexa Fluor^{®} 488 & Propidium Iodide (Invitrogen).

Les résultats sont représentés à la Figure 4. Contrairement aux cellules traitées avec le G418, les cellules traitées avec les deux concentrations de DAP montrent un taux de croissance toujours positif malgré un ralentissement de la croissance en particulier à 25 µM (Figure 4A). Ce résultat indique que le nombre de cellules en division est supérieur au nombre de cellules en arrêt du cycle cellulaire ou mortes.

De plus, contrairement aux puits cultivés en présence de G418 dont le pourcentage de cellules apoptotiques s'élève à plus de 30% à partir du 4ème jour de culture, dans les puits traités avec de la DAP, le taux ne dépassent pas les 15% (Figure 4B).

Ces résultats montrent que la DAP induit un léger ralentissement du cycle cellulaire mais pas de toxicité significative.

### 5. La DAP n'induit pas d'inhibition du NMD

Afin d'étudier comment la DAP permet de corriger les mutations non-sens UGA, il a été recherché si cette molécule pouvait inhiber le NMD. Le niveau d'un ARNm porteur d'une mutation non-sens UGA (ARNm p53 dans des cellules Calu-6) a été mesuré en absence ou en présence de DAP.

Les cellules Calu-6 ont été cultivées en présence de DAP (6,25 µM ou 25 µM), du G418 (600 µM) ou du DMSO comme contrôle négatif pendant 24 heures. Les ARN ont été purifiés avec du RNazol (MRC) et soumis à une RT-PCR comme décrit dans Gonzalez-Hilarion *et al.* (2012). Les ADNc résultants ont ensuite été amplifiés après 35 cycles de PCR en utilisant les amorces p53 sens (5'-ATGTGCTCAAGACTGGCGC-3' ; SEQ ID NO : 1), p53 anti-sens (5'-GACAGCATCAAATCATCC-3' ; SEQ ID NO : 2), GAPDH sens (5'-CATTGACCTCAACTACATGG-3' ; SEQ ID NO : 3), GAPDH anti-sens (5'-GCCATGCCAGTGAGCTTCC-3' ; SEQ ID NO : 4).

Les résultats montrent que la DAP n'est pas capable de stabiliser un ARNm porteur d'un codon stop prématuré, démontrant ainsi que la DAP n'est pas un inhibiteur de NMD (Figure 5).

### 6. La protéine translue par la DAP est fonctionnelle

Dans le but de démontrer que la protéine translue en présence de DAP est fonctionnelle, l'expression de deux gènes cibles du facteur de transcription p53, p21 et Noxa, a été mesurée dans les cellules Calu-6. L'augmentation de leur expression observée indique que la protéine p53 synthétisée est capable d'activer les gènes cibles et est donc fonctionnelle.

Comme décrit précédemment, les cellules Calu-6 ont été cultivées en présence de DAP (6,25 µM ou 25 µM), du G418 (600 µM) ou du DMSO comme contrôle négatif pendant 20 heures. Les ARN ont été purifiés avec du RNazol (MRC) et soumis à une RT-PCR comme décrit dans Gonzalez-Hilarion *et al.* (2012). Les ADNc résultants ont ensuite été amplifiés après 35 cycles de PCR en utilisant les amorces de l'ARNm p21 (sens: 5'-GGAAGACCATGTGGACCTGT-3' ; SEQ ID NO : 5 ; anti-sens 5'-GACAAGTGGGGAGGAGGAAG-3' ; SEQ ID NO : 6) ; ARNm GADD45 (sens: 5'-GGAGGAGGAGGATGACATCG-3' ; SEQ ID NO : 7 ; anti-sens 5'-GCTTGCAGTCAGTCTCACTC-3' ; SEQ ID NO : 8) ; ARNm NOXA (sens: 5'-CAGAGCTGGAAGTCGAGTGT-3' ; SEQ ID NO : 9 ; anti-sens 5'-AGGAGTCCCCTCATGCAAGT-3' ; SEQ ID NO : 10).

Les résultats présentés à la Figure 6 montrent que dès 6,25 µM de DAP, l'expression des gènes cibles de p53 augmente, indiquant que la protéine p53 synthétisée dans ces cellules en présence de DAP est fonctionnelle. On observe que le G418 permet lui aussi une expression de p53 fonctionnelle mais moins importante que pour la DAP. Ceci suggère que l'acide aminé ou les acides aminés incorporé(s) en présence du G418 à la position de la mutation non-sens est (sont) moins compatibles avec la fonction de la protéine p53 que lorsque c'est la DAP qui induit la translecture.

### 7. Test de mutations compensatoires chez la bactérie

### Matériel et méthode

Les souches bactériennes utilisées dans ce test sont les souches de *Salmonella typhimurium* TA1535, TA1537, TA98, TA100 et TA102, fournies par Moltox (Molecular Toxicology, INC, Boone, NC 28607, USA) ou par "Culture Collections of Public Health England (Porton Down, Salisbury SP4 0JG, UK).

Le test a été fait en absence ou en présence d'une solution métabolique appelée mélange S9 (mélange S9 de foie de rat traité à l'aroclor. La concentration de la fraction S9 dans le mélange S9 est de 10%). La composition du mélange S9 est donnée au Tableau 1 ci-dessous.

**Tableau 1 : Composition de la solution d'activation métabolique S9**

| Ingrédient | Concentration finale |
|---|---|
| Glucose-6-phosphate | 5 mM |
| NADP | 4 mM |
| KCl | 33 mM |
| MgCl₂ | 8 mM |
| Tampon Sodium phosphate PH 7.4 | 100 mM |
| Fraction S9 (Molecular Toxicology, INC, Boone, NC 28607, USA) | 10% (v/v) |
| Eau | à volume |

Les concentrations finales qui ont été utilisées sont 0,5 ; 1,4 ; 4,1 ; 12,3 ; 37 ; 111,1 ; 333,3 et 1000 µg/puits pour chacune des souches bactériennes et en présence ou en absence de mélange S9. Le solvant utilisé est le dimethylsulfoxide (DMSO), le volume de la solution contenant la molécule à tester ou de DMSO seul est de 25 µl par puits.

Les contrôles positifs utilisés sont :
Pour les tests réalisés en présence de mélange S9, la 2-anthranine à 1 µg/puits pour les souches TA98, TA100 et TA1535 ou 5 µg/puits pour les souches TA102 ou TA1537.

Pour les tests réalisés en absence de mélange S9, différents contrôles positifs ont été utilisés : le 2-nitrofluorène à 0,25 µg/puits pour la souche TA98, l'azide de sodium à 1 µg/puits pour la souche TA100 ou à 0,5 µg/puits pour la souche TA1535, la mitomycine C à 0,25 µg/puits pour la souche TA102 ou la 9-aminocridine à 25 µg/puits pour la souche TA1537.

Les expériences ont été faites une fois en triplicat.

Le traitement en absence ou en présence de mélange S9 s'est fait en l'ajoutant directement dans les puits.

### Résultats

Les résultats sont présentés dans les Tableaux 2 et 3. Aucun précipité n'a été observé dans les puits lors du comptage des révertants pour aucune dose testée, dans aucune souche et que ce soit en présence ou en absence de mélange S9.

**Tableau 2 : Test principal sans activation métabolique - méthode d'incorporation directe dans le puits**

| souche | composé | dose (µg/puit) | moyenne des révertants par puit | écart-type | rapport traité/solvan t | révertant individuel par colonies |
|---|---|---|---|---|---|---|
| | | | | | | |
| **TA1535** | 2,6 DAP | 1000,0 | 0,0 | 0,0 | 0,0 | 0,0,0 |
| | | 333,3 | 3,3 | 1,2 | 1,3 | 4,2,4 |
| | | 111,1 | 3,7 | 1,5 | 1,4 | 5,4,2 |
| | | 37,0 | 1,3 | 0,6 | 0,5 | 1,2,1 |
| | | 12,3 | 4,0 | 3,0 | 1,5 | 4,7,1 |
| | | 4,1 | 2,3 | 3,2 | 0,9 | 0,6,1 |
| | | 1,4 | 2,3 | 1,5 | 0,9 | 1,2,4 |
| | | 0,5 | 3,3 | 4,9 | 1,3 | 0,9,1 |
| | DMSO | | 2,7 | 1,2 | | 2,4,2 |
| | | | | | | |
| **TA1537** | 2,6 DAP | 1000,0 | 0,0 | 0,0 | 0,0 | 0,0,0 |
| | | 333,3 | 1,3 | 0,6 | 0,5 | 2,1,1 |
| | | 111,1 | 3,0 | 3,5 | 1,1 | 1,1,7 |
| | | 37,0 | 3,3 | 2,9 | 1,3 | 5,0,5 |
| | | 12,3 | 2,3 | 2,3 | 0,9 | 5,1,1 |
| | | 4,1 | 1,3 | 2,3 | 0,5 | 4,0,0 |
| | | 1,4 | 1,7 | 0,6 | 0,6 | 2,1,2 |
| | | 0,5 | 1,3 | 2,3 | 0,5 | 0,0,4 |
| | DMSO | | 2,7 | 2,3 | | 4,4,0 |
| | | | | | | |
| **TA98** | 2,6 DAP | 1000,0 | 3,0 | 2,6 | 0,4 | 2,6,1 |
| | | 333,3 | 7,3 | 4,2 | 0,9 | 6,4,12 |
| | | 111,1 | 6,0 | 4,6 | 0,8 | 5,2,11 |
| | | 37,0 | 5,3 | 2,9 | 0,7 | 7,7,2 |
| | | 12,3 | 8,3 | 2,9 | 1,0 | 5,10,10 |
| | | 4,1 | 9,3 | 2,1 | 1,2 | 11,7,10 |
| | | 1,4 | 11,0 | 1,7 | 1,4 | 12,12,9 |
| | | 0,5 | 10,7 | 0,6 | 1,3 | 11,10,11 |
| | DMSO | | 8,0 | 3,5 | | 12,6,6 |
| | | | | | | |
| **TA100** | 2,6 DAP | 1000,0 | 0,0 | 0,0 | 0,0 | 0,0,0 |
| | | 333,3 | 28,3 | 16,2 | 0,7 | 19,19,47 |
| | | 111,1 | 36,3 | 8,1 | 0,9 | 42,27,40 |
| | | 37,0 | 43,7 | 6,8 | 1,0 | 46,36,49 |
| | | 12,3 | 40,7 | 5,5 | 1,0 | 47,38,37 |
| | | 4,1 | 42,0 | 8,7 | 1,0 | 32,47,47 |
| | | 1,4 | 44,3 | 4,0 | 1,1 | 42,49,42 |
| | | 0,5 | 42,0 | 5,6 | 1,0 | 48,41,37 |
| | DMSO | | 42,0 | 7,9 | | 48,33,45 |
| | | | | | | |
| **TA102** | 2,6 DAP | 1000,0 | 3,3 | 3,2 | 0,1 | 2,7,1 |
| | | 333,3 | 50,0 | 4,6 | 1,5 | 51,45,54 |
| | | 111,1 | 54,3 | 16,3 | 1,6 | 47,43,73 |
| | | 37,0 | 42,3 | 6,0 | 1,2 | 36,43,48 |
| | | 12,3 | 35,0 | 2,0 | 1,0 | 33,37,35 |
| | | 4,1 | 37,0 | 2,6 | 1,1 | 36,35,40 |
| | | 1,4 | 34,3 | 5,9 | 1,0 | 41,30,32 |
| | | 0,5 | 32,0 | 5,6 | 0,9 | 37,26,33 |
| | DMSO | | 34,3 | 3,2 | | 33,32,38 |
| | | | | | | |
| **TA1535** | NaN3 | 0,5 | 206,7 | 10,3 | 77,5 | 218,198,20 4 |
| **TA1537** | 9AA | 25,0 | 553,7 | 65,2 | 207,6 | 583,599,47 9 |
| **TA98** | 2NF | 0,25 | 63,7 | 4,5 | 8,0 | 64,59,68 |
| **TA100** | NaN3 | 1,0 | 232,3 | 20,7 | 5,5 | 210,236,25 1 |
| **TA102** | MMC | 0,25 | 243,3 | 11,7 | 7,1 | 241,233,25 6 |

Abréviations des contrôles positifs :
NaN3 azoture de sodium
9AA 9-Aminoacrinidine
2NF 2-nitrofluorène
MMC Mitomycine C

**Tableau 3 : Test principal avec activation métabolique - méthode d'incorporation directe dans le puits**

| souche | composé | dose (µg/puit) | moyenne des révertants par puit | écart-type | rapport traité/solvant | révertant individuel par colonies |
|---|---|---|---|---|---|---|
| | | | | | | |
| **TA1535** | 2,6 DAP | 1000,0 | 0,0 | 0,0 | 0,0 | 0,0,0 |
| | | 333,3 | 14,7 | 5,0 | 4,4 | 14,10,20 |
| | | 111,1 | 13,3 | 2,1 | 4,0 | 11,14,15 |
| | | 37,0 | 8,3 | 2,1 | 2,5 | 10,9,6 |
| | | 12,3 | 4,0 | 2,0 | 1,2 | 6,4,2 |
| | | 4,1 | 3,3 | 2,9 | 1,0 | 5,5,0 |
| | | 1,4 | 2,0 | 2,0 | 0,6 | 4,2,0 |
| | | 0,5 | 3,0 | 1,7 | 0,9 | 4,4,1 |
| | DMSO | | 3,3 | 2,1 | | 1,4,5 |
| | | | | | | |
| **TA1537** | 2,6 DAP | 1000,0 | 0,0 | 0,0 | 0,0 | 0,0,0 |
| | | 333,3 | 0,7 | 0,6 | 0,3 | 1,1,0 |
| | | 111,1 | 4,0 | 0,0 | 1,5 | 4,4,4 |
| | | 37,0 | 3,7 | 1,5 | 1,4 | 5,2,4 |
| | | 12,3 | 4,0 | 2,0 | 1,5 | 6,4,2 |
| | | 4,1 | 2,0 | 0,0 | 0,8 | 2,2,2 |
| | | 1,4 | 1,7 | 0,6 | 0,6 | 2,1,2 |
| | | 0,5 | 1,3 | 0,6 | 0,5 | 1,2,1 |
| | DMSO | | 2,7 | 1,2 | | 2,2,4 |
| | | | | | | |
| **TA98** | 2,6 DAP | 1000,0 | 6,3 | 2,5 | 0,9 | 6,9,4 |
| | | 333,3 | 10,0 | 1,0 | 1,4 | 9,11,10 |
| | | 111,1 | 13,0 | 2,6 | 1,9 | 10,14,15 |
| | | 37,0 | 9,3 | 0,6 | 1,3 | 9,10,9 |
| | | 12,3 | 9,3 | 4,0 | 1,3 | 7,7,14 |
| | | 4,1 | 7,7 | 3,2 | 1,1 | 10,4,9 |
| | | 1,4 | 6,7 | 4,9 | 1,0 | 10,1,9 |
| | | 0,5 | 12,3 | 5,5 | 1,8 | 15,6,16 |
| | DMSO | | 7,0 | 5,0 | | 7,12,2 |
| | | | | | | |
| **TA100** | 2,6 DAP | 1000,0 | 0,0 | 0,0 | 0,0 | 0,0,0 |
| | | 333,3 | 53,7 | 6,7 | 1,3 | 57,46,38 |
| | | 111,1 | 51,3 | 14,7 | 1,3 | 68,46,40 |
| | | 37,0 | 49,0 | 4,4 | 1,2 | 47,46,54 |
| | | 12,3 | 53,7 | 8,0 | 1,3 | 53,46,62 |
| | | 4,1 | 49,0 | 3,5 | 1,2 | 53,47,47 |
| | | 1,4 | 41,0 | 11,5 | 1,0 | 54,32,37 |
| | | 0,5 | 43,3 | 7,6 | 1,1 | 40,52,38 |
| | DMSO | | 40,0 | 18,2 | | 28,31,61 |
| | | | | | | |
| **TA102** | 2,6 DAP | 1000,0 | 18,0 | 3,5 | 0,4 | 20,20,14 |
| | | 333,3 | 69,3 | 8,5 | 1,5 | 78,61,69 |
| | | 111,1 | 44,0 | 10,6 | 1,0 | 56,36,40 |
| | | 37,0 | 48,3 | 3,1 | 1,1 | 49,45,51 |
| | | 12,3 | 47,3 | 4,5 | 1,1 | 52,43,47 |
| | | 4,1 | 41,3 | 10,1 | 0,9 | 53,36,35 |
| | | 1,4 | 41,7 | 3,5 | 0,9 | 45,38,42 |
| | | 0,5 | 42,0 | 13,1 | 0,9 | 36,33,57 |
| | DMSO | | 45,0 | 8,7 | | 51,35,49 |
| | | | | | | |
| **TA1535** | 2AM | 1,0 | 137,7 | 11,7 | 41,3 | 148,125,140 |
| **TA1537** | 2AM | 5,0 | 36,0 | 5,3 | 13,5 | 40,38,30 |
| **TA98** | 2AM | 1,00 | 686,7 | 69,7 | 98,1 | 767,642,651 |
| **TA100** | 2AM | 1,0 | 696,0 | 19,2 | 17,4 | 705,674,709 |
| **TA102** | 2AM | 5,00 | 91,0 | \| 3,6 \| | 2,0 | 95,88,90 |

| | | | | | | |
|---|---|---|---|---|---|---|
| Abréviations des contrôles positifs : 2AM : 2-Anthramine | | | | | | |

Une forte toxicité (révélée par une diminution de révertants) n'est observée en présence et en absence de mélange S9 qu'à la dose de 1000 µg/puits pour toutes les souches sauf la souche TA98 qui ne montre une toxicité de la molécule qu'en présence de mélange S9. Par ailleurs, la souche TA1537 révèle une toxicité de la molécule dès la concentration de 333,3 µg/puits (soit environ 90 mM) et uniquement en présence du mélange S9.

Concernant des effets génotoxiques de la DAP, des augmentations du nombre de révertants n'ont été observés que dans la souche TA1535 et uniquement en présence du mélange S9. Ces augmentations sont supérieures au seuil de réponse positive dans cette souche (c'est-à-dire avec un rapport pouvant atteindre 4,4), observées aux concentrations de 111,1 et 333,3 µg/puits et suivent un effet dose. De plus, les moyennes et la plupart des valeurs individuelles obtenues pour le nombre de révertants pour les doses allant de 37 à 333,3 µg/puits sont supérieures aux valeurs obtenues avec le DMSO seul. Ces résultats permettent de conclure à un effet génotoxique de la DAP dans la souche TA1535.

Aucune autre augmentation de révertants significative n'a été observée pour les autres souches dans cette étude.

Il est à noter qu'une augmentation significative du nombre de révertants a été observée avec les contrôles positifs en présence et en absence du mélange S9 par rapport au DMSO seul validant les conditions expérimentales de l'étude.

En conclusion, dans les conditions expérimentales de cette étude, la 2,6-DAP présente une activité mutagène uniquement sur la souche TA1535 en présence d'une solution métabolique de foie de rat.

### 8. Test in vitro des micronoyaux dans les cellules murines L5178Y TK^{+/-} de lymphomes Matériel et méthode

Les cellules utilisées sont les cellules L5178Y TK^{+/-} obtenues via ATCC (American Type Culture Collection, Manassas, USA) par l'intermédiaire de Biovalley (Marne-la-vallée, France) pour l'induction de micronoyaux. Le test a été fait en absence ou en présence du mélange métabolique S9 dans lequel la fraction S9 (Moltox ; Molecular Toxicology, INC, Boone, NC 28607, USA) représente 2% du milieu de culture. La composition du mélange S9 est donnée dans le Tableau 4 ci-dessous.

**Tableau 4 : Composition du mélange S9**

| Ingrédient | Volume(s) |
|---|---|
| Glucose-6-phosphate (180mg/mL) | 1 |
| NADP (25mg/mL) | 1 |
| KCl (150mM) | 1 |
| Fraction S9 (concentration finale en S9mix 40% (v/v)) | 2 |

Dans le test avec activation métabolique, le milieu de culture est supplémenté avec 5% de mélange S9, ainsi la concentration finale de S9 dans le milieu traité est de 2%.

Les doses de DAP testées sont 0,01 ; 0,02 ; 0,04 ; 0,07 ; 0,15 ; 0,29 ; 0,58 ; 1,17 ; 2,33 et 4,66 mM. La concentration maximale a été déterminée par la solubilité de la 2,6-DAP dans le DMSO et les contraintes expérimentales de volume applicable de solution à tester dans le milieu de culture. Pour toutes les conditions expérimentales, le DMSO utilisé comme solvant de la 2,6-DAP ne représentera que 1% (v/v) du milieu de culture.

Les concentrations retenues comme ne présentant pas de cytotoxicité de la 2,6-DAP, pour les analyses génotoxiques sont 0,02, 0,04 et 0,07 mM en absence de mélange S9, et 0,04, 0,29 et 0,58 mM en présence de mélange S9.

Les cellules ont été traitées 24 heures en absence de mélange S9, ou traitées 3 heures suivies de 24 heures de culture sans traitement lorsque le mélange S9 était présent dans le milieu de culture.

Les contrôles positifs utilisés sont la mitomycine C à la concentration finale de 1 µg/ml en absence de mélange S9 ou le cyclophosphamide à la concentration finale de 6 µg/ml en présence de mélange S9.

L'expérience a été faite une fois et 1000 cellules mononuclées ont été analysées par culture. Trois doses de la 2,6-DAP, du DMSO seul ou des contrôles positifs en présence et en absence de mélange S9 ont été analysées.

L'évaluation de la cytotoxicité a été déterminée en mesurant le doublement de la population cellulaire à la fin de l'expérience.

### Résultats

Les résultats sont indiqués dans le Tableau 5 ci-après.

**Tableau 5 : Résultats obtenus avec et sans mélange S9 mix**

| Conditions de traitement | Doses (nM) | DP (% de la substance contrôle) | Nombre de cellules analysées par culture | écart-type | rapport traité/solvant |
|---|---|---|---|---|---|
| | 0 | 100 | 1000 | 1 | a) |
| Test sans S9 mix | 0,01 | 91 | | | |
| | 0,02 | 121 | 1000 | 2 | 2,0 |
| | 0,04 | 78 | 1000 | 3 | 3,0 |
| | 0,07 | 97 | 1000 | 1 | 1,0 |
| | 0,15 | 21 | | | |
| | 0,29 | # | | | |
| | 0,58 | # | | | |
| | 1,17 | # | | | |
| | 2,33 | # | | | |
| | 4,66 | # | | | |
| | MMC (µg/mL) | | 1000 | 100 | 100,0 *** |
| | 0 | 100 | 1000 | 1 | |
| Test avec S9 mix | 0,01 | 76 | | | |
| | 0,02 | 89 | | | |
| | 0,04 | 91 | 1000 | 0 | 0,0 |
| | 0,07 | 74 | | | |
| | 0,15 | 76 | | | |
| | 0,29 | 73 | 1000 | 1 | 1,0 |
| | 0,58 | 54 | 1000 | 0 | 0,5 |
| | 1,17 | 35 | | | |
| | 2,33 | 44 | | | |
| | 4,66 | 40 | | | |
| CPA (6 µg/mL) | | | 1000 | 60 | 60,0 *** |

| | | | | | |
|---|---|---|---|---|---|
| 0 : contrôle (DMSO) ; MMC : Mitocyne C ; CPA : Cyclophosphamide ; DP : doublement delà population ; Statistiques : 2*2 table de contingence ; *** : p < 0,001 ; # : la concentration cellulaire à la fin du traitement est inférieure à celle du début du traitement ; a) : données brutes obtenues avec le contrôle est égal à 0, mais il a été modifié à 1 pour permettre le calcul des ratios. | | | | | |

La 2,6-DAP montre une cytotoxicité sévère aux doses supérieures à 0,15 mM en absence de mélange S9 puisque le doublement de la population est diminué de 79 à 100%. En présence de mélange S9, une légère cytotoxicité apparait aux doses supérieures à 0,07 mM puisque le doublement de la population est diminué de 24 à 64,6%.

Aucune augmentation significative de la fréquence de cellules contenant des micronoyaux n'a été observée en comparaison du DMSO seul, en présence ou en absence du mélange S9 indiquant aucun effet génotoxique de la 2,6-DAP. Il est à noter que la fréquence de cellules avec des micronoyaux augmente significativement en présence des contrôles positifs en présence ou en absence de mélange S9 validant ainsi les conditions expérimentales du test.

En conclusion, dans les conditions expérimentales de l'étude, la 2,6-DAP n'induit aucun dommage chromosomique ou de la machinerie cellulaire impliquée dans la division cellulaire dans des cellules somatiques de mammifères L5178Y TK+/- en absence ou en présence de solution métabolique de rat.

### 9. Mesure de l'EC50

Les cellules HeLa sont transfectées avec la construction luciférase portant une mutation non-sens UGA décrite dans l'exemple 1 en utilisant de la lipofectamine 3000 selon le protocole du fabricant (Lifetechnologies). Le jour suivant, les cellules sont réparties en plaque 96 puits et la DAP à tester est ajoutées à différentes concentrations avant une incubation de 24 heures. La DAP a été ajoutée à une concentration de 25 µM, 100 µM, 300 µM, ou 600 µM. Le substrat SteadyLite plus (Perkin Elmer) est ensuite ajouté au milieu de culture et les plaques sont lues au luminomètre Tristar (Berthold). Chaque puits est lu sur 10 secondes et la plaque est lue deux fois.

L'efficacité de correction de la mutation non-sens UGA est représentée par l'activité luciférase (Figure 7). L'efficacité maximum de la correction de la mutation non-sens UGA est obtenue avec une concentration de DAP de 100 µM. L'EC50 correspond à la concentration de DAP avec laquelle 50% de l'activité luciférase maximum. L'EC50 de la DAP est de 54 µM.

### 10. Mesure de la cytotoxicité

Les cellules Hela sont mises en culture dans des plaques 96 puits en présence de différentes concentrations de DAP (0.39, 0.78, 1.56, 6.25, 25, 100, 300, 600, 1200, 2400 µM). Le DMSO représente le contrôle négatif. La staurosporine (STS) a été ajoutée comme contrôle positif. Après 20 heures de culture à 37°C et 5% de CO₂, les cellules sont ramenées à température ambiante pendant 5 minutes. 100 µL de AK détection reagent (ToxiLight^{™} bioassay kit, Lonza) sont ajoutés dans chacun des puits afin de mesurer l'activité de l'adénylate kinase, une enzyme larguée dans le milieu extracellulaire lorsque les cellules sont mortes. Les cellules sont incubées 5 minutes avec le réactif avant que les plaques soient lues au luminomètre Tristar (Berthold). Aucune toxicité n'est observée dans les différentes concentrations de DAP utilisées en comparaison au traitement à la staurosporine (Figure 8).

### 11. Synergie avec la clitocine

Les cellules HeLa sont transfectées avec la construction luciférase portant une mutation non-sens UGA décrite dans l'exemple 1 en utilisant de la lipofectamine 3000 selon le protocole du fabricant (Lifetechnologies). Le jour suivant, les cellules sont réparties en plaque 96 puits et incubées pendant 20 heures en présence de concentrations croissantes de DAP et de clitocine en combinaison (0-600 µM). L'activité luciférase est mesurée en ajoutant le substrat SteadyLite plus (Perkin Elmer) au milieu de culture et les plaques sont lues au luminomètre Tristar (Berthold). La plaque est lue deux fois.

Un effet synergique de la DAP et de la clitocine est observé (Figure 9), l'effet observé pour des combinaisons de DAP et de clitocine étant supérieur à l'effet additif attendu. A titre d'exemple, l'efficacité de correction de la mutation non-sens UGA observée en présence de 1,56 µM de clitocine et 25 µM de DAP (luminescence mesurée: 250 000) est supérieure à l'efficacité qui serait attendue pour un effet additif du même mélange de clitocine et de DAP (luminescence mesurée pour la clitocine à 1,56 µM: 100 000; luminescence mesurée pour la DAP seule à 25 µM: 50 000; luminescence attendue pour un effet additif de la clitocine et de la DAP à ces concentrations: 150 000). L'efficacité maximale de correction de la mutation non-sens UGA est obtenue en combinant la DAP à 100 µM et la clitocine à 0,39 µM.

## Revendications

1. 2,6-diaminopurine (DAP) pour son utilisation dans le traitement d'une maladie due une mutation non-sens dans un gène conduisant à l'introduction prématurée d'un codon stop UGA.

2. DAP pour son utilisation selon la revendication 1, **caractérisée en ce que** ladite maladie est choisie parmi la mucoviscidose due à ladite mutation non-sens, les dystrophies musculaires dues à ladite mutation non-sens, l'hémophilie due à ladite mutation non-sens, la béta-thalassémie due à ladite mutation non-sens, la rétinite pigmentaire due à ladite mutation non-sens, les mucopolysaccharidoses dues à ladite mutation non-sens, l'amyotrophie spinale due à ladite mutation non-sens.

3. DAP pour son utilisation selon l'une quelconque des revendications 1 à 2, **caractérisée en ce qu'**elle est destinée à être administrée à un sujet en combinaison avec un composé possédant une activité de translecture choisi dans le groupe constitué par la 6-amino-5-nitro-4-(α-D-ribofuranosylamino)-pyrimidine, la 6-amino-5-nitro-4-(β-D-ribofuranosylamino)-pyrimidine, l'ataluren, la gentamycine, la geneticine, la paromomycine et les dérivés de la paromomycine tels que NB30; NB54; NB74; NB84, l'amikacine, la tobramycine, la pyramicine et les dérivés de la pyramycine tels que TC001 ; TC003; TC007; TC032, la kanamycine et les dérivés de la kanamycine tels que JL022; JL023, l'amlexanox, le RTC 13, le RTC 14, le 3-(2-4E(l,l dimethyl propyl)-phenoxy- acetylamino)-benzoic acid, le 3-(2-(4-isopropyl-3-methyl-phenoxy)-acetylamino)-benzoic acid, la negamycine, la tylosine, la josamycine, la spiramycine, et le 3-(2-(4-tert-butyl- phenoxy)-acetylamino)-benzoic acid.

4. Composition pharmaceutique comprenant la DAP et un excipient pharmaceutiquement acceptable pour son utilisation pour le traitement d'une maladie due à une mutation non-sens dans un gène conduisant à l'introduction prématurée d'un codon stop UGA.

5. Composition pharmaceutique pour son utilisation selon la revendication 4, **caractérisée en ce que** ladite maladie est choisie parmi la mucoviscidose due à ladite mutation non-sens, les dystrophies musculaires dues à ladite mutation non-sens, l'hémophilie due à ladite mutation non-sens, la béta-thalassémie due à ladite mutation non-sens, la rétinite pigmentaire due à ladite mutation non-sens, les mucopolysaccharidoses dues à ladite mutation non-sens, l'amyotrophie spinale due à ladite mutation non-sens.

6. Composition pharmaceutique pour son utilisation selon l'une quelconque des revendications 4 à 5, **caractérisée en ce qu'**elle est destinée à être administrée à un sujet en combinaison avec un composé possédant une activité de translecture choisi dans le groupe constitué par la 6-amino-5-nitro-4-(α-D-ribofuranosylamino)-pyrimidine, la 6-amino-5-nitro-4-(β-D-ribofuranosylamino)-pyrimidine, l'ataluren, la gentamycine, la geneticine, la paromomycine et les dérivés de la paromomycine tels que B30; NB54; NB74; NB84, l'amikacine, la tobramycine, la pyramicine et les dérivés de la pyramycine tels que TCOOl ; TC003; TC007; TC032, la kanamycine et les dérivés de la kanamycine tels que JL022; JL023, l'amlexanox, le RTC 13, le RTC 14, le 3-(2-4E(l,l dimethyl propyl)-phenoxy-acetylamino)-benzoic acid, le 3-(2-(4-isopropyl-3-methyl-phenoxy)-acetylamino)-benzoic acid, la negamycine, la tylosine, la josamycine, la spiramycine, et le 3-(2-(4-tert-butyl-phenoxy)-acetylamino)-benzoic acid.

7. Composition comprenant la DAP et un composé possédant une activité de translecture choisi dans le groupe constitué par la 6-amino-5-nitro-4-(α-D-ribofuranosylamino)-pyrimidine, la 6-amino-5-nitro-4-(β-D-ribofuranosylamino)-pyrimidine, l'ataluren, la gentamycine, la geneticine, la paromomycine et les dérivés de la paromomycine tels que NB30; NB54; NB74; B84, l'amikacine, la tobramycine, la pyramicine et les dérivés de la pyramycine tels que TCOOl ; TC003; TC007; TC032, la kanamycine et les dérivés de la kanamycine tels que JL022; JL023, l'amlexanox, le RTC 13, le RTC 14, le 3-(2-4E(l,l dimethyl propyl)-phenoxy-acetylamino)-benzoic acid, le 3-(2-(4-isopropyl-3-methyl-phenoxy)-acetylamino)-benzoic acid, la negamycine, la tylosine, la josamycine, la spiramycine, et le 3-(2-(4-tert-butyl-phenoxy)-acetylamino)-benzoic acid, pour leur utilisation simultanée, séparée ou séquentielle pour le traitement d'une maladie due à une mutation non-sens dans un gène conduisant à l'introduction prématurée d'un codon stop UGA.

## Patentansprüche

1. 2,6-Diaminopurin (DAP) zur Verwendung bei der Behandlung einer Krankheit aufgrund einer Nonsense-Mutation in einem Gen, die zur vorzeitigen Einführung eines Stopcodons UGA führt.

2. DAP zur Verwendung nach Anspruch 1, **dadurch gekennzeichnet, dass** die Krankheit ausgewählt ist aus Mukoviszidose aufgrund der Nonsense-Mutation, Muskeldystrophien aufgrund der Nonsense-Mutation, Hämophilie aufgrund der Nonsense-Mutation, Beta-Thalassämie aufgrund der Nonsense-Mutation, Retinitis pigmentosa aufgrund der Nonsense-Mutation, Mukopolysaccharidosen aufgrund der Nonsense-Mutation, spinaler Muskelatrophie aufgrund der Nonsense-Mutation.

3. DAP zur Verwendung nach einem der Ansprüche 1 bis 2, **dadurch gekennzeichnet, dass** es dazu bestimmt ist, einem Probanden in Kombination mit einer Verbindung verabreicht zu werden, die eine Durchleseaktivität besitzt, ausgewählt aus der Gruppe, bestehend aus 6-Amino-5-nitro-4-(α-D-ribofuranosylamino)pyrimidin, 6-Amino-5-nitro-4-(β-D-ribofuranosylamino)pyrimidin, Ataluren, Gentamycin, Geneticin, Paromomycin und den Derivaten von Paromomycin wie NB30; NB54; NB74; NB84, Amikacin, Tobramycin, Pyramicin und den Derivaten von Pyramycin wie TC001; TC003; TC007; TC032, Kanamycin und den Derivaten von Kanamycin wie JL022; JL023, Amlexanox, RTC 13, RTC 14, 3-(2-4E(I,I-Dimethylpropyl)phenoxyacetylamino)benzoesäure, 3-(2-(4-Isopropyl-3-methylphenoxy)acetylamino)benzoesäure, Negamycin, Tylosin, Josamycin, Spiramycin und 3-(2-(4-tert-Butylphenoxy)acetylamino)benzoesäure.

4. Pharmazeutische Zusammensetzung, umfassend DAP und ein pharmazeutisch verträgliches Hilfsmittel zur Verwendung für die Behandlung einer Krankheit aufgrund einer Nonsense-Mutation in einem Gen, die zur vorzeitigen Einführung eines Stopcodons UGA führt.

5. Pharmazeutische Zusammensetzung zur Verwendung nach Anspruch 4, **dadurch gekennzeichnet, dass** die Krankheit ausgewählt ist aus Mukoviszidose aufgrund der Nonsense-Mutation, Muskeldystrophien aufgrund der Nonsense-Mutation, Hämophilie aufgrund der Nonsense-Mutation, Beta-Thalassämie aufgrund der Nonsense-Mutation, Retinitis pigmentosa aufgrund der Nonsense-Mutation, Mukopolysaccharidosen aufgrund der Nonsense-Mutation, spinaler Muskelatrophie aufgrund der Nonsense-Mutation.

6. Pharmazeutische Zusammensetzung zur Verwendung nach einem der Ansprüche 4 bis 5, **dadurch gekennzeichnet, dass** sie dazu bestimmt ist, einem Probanden in Kombination mit einer Verbindung verabreicht zu werden, die eine Durchleseaktivität besitzt, ausgewählt aus der Gruppe, bestehend aus 6-Amino-5-nitro-4-(α-D-ribofuranosylamino)pyrimidin, 6-Amino-5-nitro-4-(ß-D-ribofuranosylamino)pyrimidin, Ataluren, Gentamycin, Geneticin, Paromomycin und den Derivaten von Paromomycin wie B30; NB54; NB74; NB84, Amikacin, Tobramycin, Pyramicin und den Derivaten von Pyramycin wie TCOOI; TC003; TC007; TC032, Kanamycin und den Derivaten von Kanamycin wie JL022; JL023, Amlexanox, RTC 13, RTC 14, 3-(2-4E(l,l-Dimethylpropyl)phenoxyacetylamino)benzoesäure, 3-(2-(4-Isopropyl-3-methylphenoxy)acetylamino)benzoesäure, Negamycin, Tylosin, Josamycin, Spiramycin und 3-(2-(4-tert-Butylphenoxy)acetylamino)benzoesäure.

7. Zusammensetzung, umfassend DAP und eine Verbindung, die eine Durchleseaktivität besitzt, ausgewählt aus der Gruppe, bestehend aus 6-Amino-5-nitro-4-(α-D-ribofuranosylamino)pyrimidin, 6-Amino-5-nitro-4-(β-D-ribofuranosylamino)pyrimidin, Ataluren, Gentamycin, Geneticin, Paromomycin und den Derivaten von Paromomycin wie NB30; NB54; NB74; B84, Amikacin, Tobramycin, Pyramicin und den Derivaten von Pyramycin wie TCOOI ; TC003; TC007; TC032, Kanamycin und den Derivaten von Kanamycine wie JL022; JL023, Amlexanox, RTC 13, RTC 14, 3-(2-4E(I,I-Dimethylpropyl)phenoxyacetylamino)benzoesäure, 3-(2-(4-Isopropyl-3-methyl-phenoxy)acetylamino)benzoesäure, Negamycin, Tylosin, Josamycin, Spiramycin und 3-(2-(4-tert-Butylphenoxy)acetylamino)benzoesäure, für deren gleichzeitige, getrennte oder aufeinander folgende Verwendung für die Behandlung einer Krankheit aufgrund einer Nonsense-Mutation in einem Gen, die zur vorzeitigen Einführung eines Stopcodons UGA führt.

## Claims

1. 2,6-diaminopurine (DAP) for the use thereof in the treatment of a disease due to a nonsense mutation in a gene leading to the premature introduction of a UGA stop codon.

2. DAP for the use thereof according to claim 1, **characterised in that** said disease is selected from among cystic fibrosis due to said nonsense mutation, muscular dystrophies due to said nonsense mutation, beta-thalassemia due to said nonsense mutation, retinitis pigmentosa due to said nonsense mutation, mucopolysaccharidosis due to said nonsense mutation, spinal muscular atrophy due to said nonsense mutation.

3. DAP for the use thereof according to any one of the claims 1 to 2, **characterised in that** it is intended to be administered to a subject in combination with a compound having a readthrough activity selected from the group consisting of 6-amino-5-nitro-4-(α-D-ribofuranosylamino)-pyrimidine, 6-amino-5-nitro-4-(β-D-ribofuranosylamino)-pyrimidine, ataluren, gentamicin, geneticin, paromomycin and paromomycin derivatives such as NB30; NB54; NB74; NB84, amikacin, tobramycin, pyramycine and pyramycine derivatives such as TC001; TC003; TC007; TC032, kanamycin and kanamycin derivatives such as JL022; JL023, amlexanox, RTC 13, RTC 14, 3-(2-4E(1,1 dimethyl propyl)-phenoxy-acetylamino)-benzoic acid, 3-(2-(4-isopropyl-3-methyl-phenoxy)-acetylamino)-benzoic acid, negamycin, tylosin, josamycine, spiramycin, and 3-(2-(4-tert-butyl-phenoxy)-acetylamino)-benzoic acid.

4. Pharmaceutical composition comprising DAP and a pharmaceutically-acceptable excipient for the use thereof for the treatment of a disease due to a nonsense mutation in a gene leading to the premature introduction of a UGA stop codon.

5. Pharmaceutical composition for the use thereof according to claim 4, **characterised in that** said disease is selected from among cystic fibrosis due to said nonsense mutation, muscular dystrophies due to said nonsense mutation, beta-thalassemia due to said nonsense mutation, retinitis pigmentosa due to said nonsense mutation, mucopolysaccharidosis due to said nonsense mutation, spinal muscular atrophy due to said nonsense mutation.

6. Pharmaceutical composition for the use thereof according to any one of claims 4 to 5, **characterised in that** it is intended to be administered to a subject in combination with a compound having a readthrough activity selected from the group consisting of 6-amino-5-nitro-4-(α-D-ribofuranosylamino)-pyrimidine, 6-amino-5-nitro-4-(β-D-ribofuranosylamino)-pyrimidine, ataluren, gentamicin, geneticin, paromomycin and paromomycin derivatives such as NB30; NB54; NB74; NB84, amikacin, tobramycin, pyramycine and pyramycine derivatives such as TC001; TC003; TC007; TC032, kanamycin and kanamycin derivatives such as JL022; JL023, amlexanox, RTC 13, RTC 14, 3-(2-4E(1,1 dimethyl propyl)-phenoxy-acetylamino)-benzoic acid, 3-(2-(4-isopropyl-3-methyl-phenoxy)-acetylamino)-benzoic acid, negamycin, tylosin, josamycine, spiramycin, and 3-(2-(4-tert-butyl-phenoxy)-acetylamino)-benzoic acid.

7. Composition comprising DAP and a compound having a readthrough activity selected from the group consisting of 6-amino-5-nitro-4-(α-D-ribofuranosylamino)-pyrimidine, 6-amino-5-nitro-4-(β-D-ribofuranosylamino)-pyrimidine ataluren, gentamicin, geneticin, paromomycin and paromomycin derivatives such as NB30; NB54; NB74; NB84, amikacin, tobramycin, pyramycine and pyramycine derivatives such as TC001; TC003; TC007; TC032, kanamycin and kanamycin derivatives such as JL022; JL023, amlexanox, RTC 13, RTC 14, 3-(2-4E(1,1 dimethyl propyl)-phenoxy-acetylamino)-benzoic acid, 3-(2-(-4-isopropyl-3-methyl-phenoxy)-acetylamino)-benzoic acid, negamycin, tylosin, josamycine, spiramycin, and 3-(2-(4-tert-butyl-phenoxy)-acetylamino)-benzoic acid, for the simultaneous, separate or sequential use thereof for the treatment of a disease due to a nonsense mutation of a gene leading to the premature introduction of a UGA stop codon.
